(19) Europäisches Patentamt
European Patent Office
Office européen des brevets

(11) EP 3 302 483 B1

(12) **EUROPEAN PATENT SPECIFICATION**

(45) Date of publication and mention of the grant of the patent:
**15.04.2020 Bulletin 2020/16**

(21) Application number: **16799287.4**

(22) Date of filing: **24.05.2016**

(51) Int Cl.:
*A61K 9/16* (2006.01) *A61K 9/20* (2006.01)
*A61K 31/506* (2006.01) *A61P 35/00* (2006.01)
*A61K 9/48* (2006.01) *A61K 45/06* (2006.01)

(86) International application number:
**PCT/CN2016/083098**

(87) International publication number:
**WO 2016/188399 (01.12.2016 Gazette 2016/48)**

(54) **PHARMACEUTICAL COMPOSITIONS AND USE THEREOF**

PHARMAZEUTISCHE ZUSAMMENSETZUNGEN UND VERWENDUNG DAVON

COMPOSITIONS PHARMACEUTIQUES ET UTILISATION DE CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.05.2015 PCT/CN2015/079650**

(43) Date of publication of application:
**11.04.2018 Bulletin 2018/15**

(73) Proprietor: **Hutchison Medipharma Limited**
**Shanghai 201203 (CN)**

(72) Inventors:
- **LIU, Zhongzhou**
**Shanghai 201203 (CN)**
- **FU, Chongdong**
**Shanghai 201203 (CN)**
- **SHI, Bin**
**Shanghai 201203 (CN)**

(74) Representative: **Bianchetti Bracco Minoja S.r.l.**
**Via Plinio, 63**
**20129 Milano (IT)**

(56) References cited:
**WO-A1-2011/060746**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 302 483 B1

## Description

Angiogenesis is a process wherein new blood vessels can grow from existing vasculature. That process can occur in wound healing of the body, such as the restoration of blood flow in tissue injury, for example, an injury of the hand. Excess angiogenesis, however, might be initiated under specific pathological conditions, for example tumor, AMD (age-related macular degeneration), rheumatoid arthritis, psoriasis, etc. Under such circumstances, new blood vessels may undesirably tend to provide pathological tissues with nutrition and injure the normal tissues. For example, cancer cells may enter into blood circulation through new blood vessels and invade normal tissues.

VEGF (Vascular Endothelial Growth Factor) and its receptor VEGFR-2 (also called KDR, kinase insert domain-containing receptor) can form the major pathway for the formation of new blood vessels. It has been indicated that inhibition of KDR can cause apoptosis of endothelial cells, which consequently block the angiogenesis process (Rubin M. Tuder, Chest, 2000; 117:281). Thus, KDR inhibitors can be used for the treatment of angiogenesis- related diseases.

FGF (Fibroblast Growth Factor) is a pro-angiogenesis molecule as is VEGF. During angiogenesis, VEGF is thought to be critical in the neovascularization process. The FGF (Fibroblast Growth Factor)/FGFR (Fibroblast Growth Factor Receptor) axis plays roles in functionally maturing newly formed vessels. In addition, aberrant activation of FGF family members and their cognate receptors have been found in multiple cancers, such as breast, bladder and prostate cancers. FGFR1 and its binding partners FGF1, FGF2, FGF8b and FGF17 are also elevated. In other tumor types, FGFR1 is implicated as an oncogene whose expression is increased compared with normal tissue. Therefore, blockade of FGF/FGFR signaling may be beneficial for treatment of cancers associated with FGF/FGFR activation.

Neuroendocrine tumors (NETs) are rare cancer of the hormone system, normally slow growth. NETs belong to Neuroendocrine neoplasms (NENs), which are rare tumors that arise from embryological neuroendocrine cells, have neuroendocrine biomarkers, and may produce polypeptide hormones. Since NENs have a long natural disease course with a relatively high 5-year survival rate compared to other tumors, there is a large number of surviving NENs patients. NENs occur in various organs and tissues across the body.

The World Health Organization (WHO) classified NENs into three basic types: low-grade (G1), intermediate-grade (G2) and high-grade (G3) (also known as neuroendocrine carcinoma, NEC). The first two types (G1 and G2) are collectively referred to as NETs. NETs show a mitotic rate (mitotic count ≤ 20/10 HPE) and/or a Ki67 proliferation index (≤ 20%) of tumor cells. In general, the higher the grade (i.e. the higher the mitotic count and Ki-67 proliferation index), the more aggressive the tumor will become and the poorer prognosis the patient will have. Although low and intermediate grade NETs show slow tumor progression and early NETs can be successfully treated with surgery, patients with advanced NETs are not sensitive to chemotherapy and have very limited treatment options. Thus, advanced NETs are a type of refractory tumor.

NETs arising in the pancreas can be distinguished from those arising else-where in the gastrointestinal tract. Although pancreatic neuroendocrine tumors (PNETs) were originally thought to arise from pancreatic islet cells, recent work supports the notion that the PNETs arise from stem-like nonislet ductal progenitor cell, sustaining the transition in nomenclature from islet cell carcinoma to pancreatic neuroendocrine tumor. Current medications for PNETs include somatostatin, interferon, chemotherapeutics, and molecular targeted drugs (such as Sunitinib and Everolimus). PNETs patients are not sensitive to traditional chemotherapy, and only two targeted therapies are proved to prolong the progression free survival (PFS) in advanced PNETs.

Extrapancreatic neuroendocrine neoplasms (Extrapancreatic NETs) originate in organs or tissue other than pancreas, of which gastrointestinal neuroendocrine tumors (GI-NETs), found in the stomach, duodenum, small intestines, appendix, cecum, colon and rectum, are the most common. Currently, only long-acting somatostatin analogs (SSAs; including octreotide and lanreotide) have received FDA approval for use in treating extrapancreatic NETs. However, due to their low anti-tumor activity, SSAs are mainly used in clinical studies which enroll patients with G1 grade tumors, because such tumors have a better prognosis and a slow growth rate.

The compound of Formula A ("Compound A" and "compound of formula A" are used interchangeably herein), i.e., *N*-(2-(dimethylamino) ethyl)-1-(3-((4-((2-methyl-1*H*-indol-5-yl)oxy)pyrimidin-2-yl)amino)phenyl)-methanesulfonamide, and/or a pharmaceutically acceptable salt thereof was disclosed in US Patent Application No.: 13/510,249 (the '249 application), which is a national stage of PCT/CN2010/078997, filed November 23, 2010, now issued as U.S. Patent No.: 8,658,658 (the '658 patent).

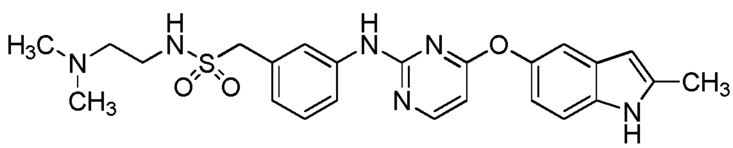

Formula A

*N*-(2-(dimethylamino)ethyl)-1-(3-((4-((2-methyl-1*H*-indol-5-yl)oxy)pyrimidin-2-yl)amino)phenyl)-methanesulfonamide

Solid-state crystalline forms I and II of the compound of Formula A, i.e., Form I *N*-(2-(dimethylamino) ethyl)-1-(3-((4-((2-methyl-1*H*-indol-5-yl)oxy)pyrimidin-2-yl)amino)phenyl)-methanesulfonamide and Form II *N-(2*-(dimethylamino) ethyl)-1-(3-((4-((2-methyl-1*H*-indol-5-yl)oxy)pyrimidin-2-yl)amino)phenyl)-methanesulfonamide, and methods of preparation thereof had been discovered and were disclosed in the '658 patent.

Disclosed herein is a the micronized compound of Formula A and/or the micronized at least one pharmaceutically acceptable salt thereof wherein the micronized Compound A and/or the micronized at least one pharmaceutically acceptable salt of Compound A has a particle size distribution (PSD) D90 value of less than or equal to 20 μm. In some embodiments, the compound of Formula A is Form I. In some embodiments, the compound of Formula A is substantially pure Form I. In some embodiments, the compound of Formula A is Form II. In some embodiments, the compound of Formula A is substantially pure Form II. In some embodiments, the compound of Formula A and/or a pharmaceutically acceptable salt thereof are micronized with a D90 value of less than or equal to 20.0 μm, such as ranging from 1.0-20.0 μm, or ranging from 2.0-12.0 μm. In some embodiments, Form I, substantially pure Form I, Form II, or substantially pure Form II is micronized with a D90 value of less than or equal to 20.0 μm, such as ranging from 1.0-20.0 μm, or ranging from 2.0-12.0 μm. In some embodiments, the D90 value ranges from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments, the D90 value ranges from 2.0 to 5.0 μm, for example, the D90 value is 3.0, 3.5, or 4.0 μm. In some embodiments, the D90 value ranges from 9.0 to 12.0 μm, for example, the D90 value is 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm. The micronized Compound A as above disclosed are used in the treatment of at least one disease responsive to FGFR1 inhibition, and/or at least one disease responsive to KDR inhibition, wherein said disease is an angiogenesis- related disease.

In some embodiments, the neuroendocrine tumors are pancreatic neuroendocrine tumors. In some embodiments, the neuroendocrine tumors are extrapancreatic neuroendocrine tumors. In some embodiments, the neuroendocrine tumors are gastrointestinal neuroendocrine tumors.

Also disclosed is a pharmaceutical composition comprising micronized Compound A , and/or micronized at least one pharmaceutically acceptable salt of Compound A, and at least one pharmaceutically acceptable excipient, wherein the micronized Compound A and/or the micronized at least one pharmaceutically acceptable salt of Compound A has a particle size distribution (PSD) D90 value of less than or equal to 20 μm for use in the treatment of at least one disease responsive to FGFR1 inhibition, and/or at least one disease responsive to KDR inhibition, wherein said disease is an angiogenesis- related disease. In some embodiments, Compound A is Form I. In some embodiments, Compound A is substantially pure Form I. In some embodiments, Compound A is Form II. In some embodiments, Compound A is substantially pure Form II. In some embodiments, Compound A, Form I, substantially pure Form I, Form II, or substantially pure Form II is micronized with a D90 value of less than or equal to 20.0 μm, such as ranging from 1.0-20.0 μm, or ranging from 2.0-12.0 μm. In some embodiments, the D90 value ranges from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments, the D90 value ranges from 2.0 to 5.0 μm, for example, the D90 value is 3.0, 3.5, or 4.0 μm. In some embodiments, the D90 value ranges from 9.0 to 12.0 μm, for example, the D90 value is 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

Also disclosed herein is a first pharmaceutical composition, comprising
micronized Compound A , and/or
micronized at least one pharmaceutically acceptable salt of Compound A, and
at least one pharmaceutically acceptable excipient wherein the micronized Compound A and/or the micronized at least one pharmaceutically acceptable salt of Compound A has a particle size distribution (PSD) D90 value of less than or equal to 20.0 μm, such as ranging from 1.0-20.0 μm, or ranging from 2.0-12.0 μm. In some embodiments, the D90 value ranges from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments, the D90 value ranges from 2.0 to 5.0 μm, for example, the D90 value is 3.0, 3.5, or 4.0 μm. In some embodiments, the D90 value ranges from 9.0 to 12.0 μm, for example, the D90 value is 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

In some embodiments of the first pharmaceutical composition, the micronized Compound A is micronized Form I. In some embodiments of the first pharmaceutical composition, the micronized Compound A is micronized substantially

pure Form I.

In some embodiments of the first pharmaceutical composition, the micronized Compound A is micronized Form II. In some embodiments of the first pharmaceutical composition, the micronized Compound A is micronized substantially pure Form II.

In some embodiments of the first pharmaceutical composition, the micronized Form I or micronized substantially pure Form I has a D90 value of less than or equal to 20.0 μm, such as ranging from 1.0-20.0 μm, or ranging from 2.0-12.0 μm. In some embodiments of the first pharmaceutical composition, the micronized Form I or micronized substantially pure Form I has a D90 value ranging from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments of the first pharmaceutical composition, the micronized Form I or micronized substantially pure Form I has a D90 value ranging from 2.0 to 5.0 μm, for example, has a D90 value as 3.0, 3.5, or 4.0 μm. In some embodiments of the first pharmaceutical composition, the micronized Form I or micronized substantially pure Form I has a D90 value ranging from 9.0 to 12.0 μm, for example, has a D90 value as 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

In some embodiments of the first pharmaceutical composition, the micronized Form II or micronized substantially pure Form II has a D90 value less than or equal to 20.0 μm, such as ranging from 1.0-20.0 μm, or ranging from 2.0-12.0 μm. In some embodiments of the first pharmaceutical composition, the micronized Form II or micronized substantially pure Form II has a D90 value ranging from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments of the first pharmaceutical composition, the micronized Form II or micronized substantially pure Form II has a D90 value ranging from 2.0 to 5.0 μm, for example, has a D90 value as 3.0, 3.5, or 4.0 μm. In some embodiments of the first pharmaceutical composition, the micronized Form II or micronized substantially pure Form II has a D90 value ranging from 9.0 to 12.0 μm, for example, has a D90 value as 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

In some embodiments of the first pharmaceutical composition, the at least one pharmaceutically acceptable excipient is chosen from diluents (such as mannitol, microcrystalline cellulose, starch, lactose, dextrin, sorbitol), disintegrants (such as sodium starch glycolate), granulation binders (such as polyvinylpyrrolidone (PVP)), and glidants (such as silicon dioxide and magnesium stearate). In some embodiments, the diluent can be present in an amount from about 35% to about 90% by weight of the composition. In some embodiments, the glidant can be present in an amount from about 0.1% to about 5% by weight of the composition. In some embodiments, the disintegrant can be present in an amount from about 0.5% to about 10% by weight of the composition. In some embodiments, the granulation binders can be present in an amount from about 0.5% to about 5% by weight of the composition. In some embodiments of the first pharmaceutical composition, the at least one pharmaceutically acceptable excipient is chosen from mannitol, microcrystalline cellulose, sodium starch glycolate, polyvinylpyrrolidone (PVP), and magnesium stearate. In some embodiments of the first pharmaceutical composition, the at least one pharmaceutically acceptable excipient is chosen from microcrystalline cellulose, sodium starch glycolate, silicon dioxide, and magnesium stearate. In some embodiments of the first pharmaceutical composition, the at least one pharmaceutically acceptable excipient is chosen from microcrystalline cellulose, sodium starch glycolate, and magnesium stearate. In some embodiments of the first pharmaceutical composition, the at least one pharmaceutically acceptable excipient is chosen from microcrystalline cellulose and magnesium stearate.

In some embodiments of the first pharmaceutical composition, the pharmaceutical composition is in form of tablet or capsule, the micronized Compound A , such as micronized Form I/substantially pure Form I or micronized Form II/substantially pure Form II, and/or micronized at least one pharmaceutically acceptable salt thereof can be present in an amount of 1, 5,10, 15, 20, 25, 50, 75, 80, 85, 90, 95, 100, 125, 150, 200, 250, 300, 400 and 500 mg in a tablet or capsule, such as in a capsule.

Also disclosed herein is a second pharmaceutical composition, comprising
micronized Compound A , and
at least one pharmaceutically acceptable excipient, wherein the particle size distribution (PSD) of the micronized Compound A has a D90 value of less than or equal to 20.0 μm, such as ranging from 1.0-20.0 μm, or ranging from 2.0-12.0 μm. In some embodiments, the D90 value ranges from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments, the D90 value ranges from 2.0 to 5.0 μm, for example, the D90 value is 3.0, 3.5, or 4.0 μm. In some embodiments, the D90 value ranges from 9.0 to 12.0 μm, for example, the D90 value is 9.5 or 10.0 μm. In a preferred embodiment, the D90 value is less than or equal to 11.0 μm or is less than

or equal to 10.0 μm. In a more preferred embodiment, the D90 value is less than or equal to 6.0 μm. In a particular embodiment, the D90 value is less than or equal to 4.0 μm.

In some embodiments of the second pharmaceutical composition, the micronized Compound A is micronized Form I. In some embodiments of the second pharmaceutical composition, the micronized Compound A is micronized substantially pure Form I.

In some embodiments of the second pharmaceutical composition, the micronized Compound A is micronized Form II. In some embodiments of the second pharmaceutical composition, the micronized Compound A is micronized substantially pure Form II.

In some embodiments of the second pharmaceutical composition, the micronized Form I or micronized substantially pure Form I has a D90 value of less than or equal to 20.0 μm, such as ranging from 1.0-20.0 μm, or ranging from 2.0-12.0 μm. In some embodiments of the second pharmaceutical composition, the micronized Form I or micronized substantially pure Form I has a D90 value ranging from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments of the second pharmaceutical composition, the micronized Form I or micronized substantially pure Form I has a D90 value ranging from 2.0 to 5.0 μm, for example, has a D90 value as 3.0, 3.5, or 4.0 μm. In some embodiments of the second pharmaceutical composition, the micronized Form I or micronized substantially pure Form I has a D90 value ranging from 9.0 to 12.0 μm, for example, has a D90 value as 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

In some embodiments of the second pharmaceutical composition, the micronized Form II or micronized substantially pure Form II has a D90 value has a D90 value of less than or equal to 20.0 μm, such as ranging from 1.0-20.0 μm, or ranging from 2.0-12.0 μm. In some embodiments of the second pharmaceutical composition, the micronized Form II or micronized substantially pure Form II has a D90 value ranging from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments of the second pharmaceutical composition, the micronized Form II or micronized substantially pure Form II has a D90 value ranging from 2.0 to 5.0 μm, for example, has a D90 value as 3.0, 3.5, or 4.0 μm. In some embodiments of the second pharmaceutical composition, the micronized Form II or micronized substantially pure Form II has a D90 value ranging from 9.0 to 12.0 μm, for example, has a D90 value as 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

In some embodiments of the second pharmaceutical composition, the at least one pharmaceutically acceptable excipient is chosen from diluents (such as mannitol, microcrystalline cellulose, starch, lactose, dextrin, sorbitol), disintegrants (such as sodium starch glycolate), granulation binders (such as polyvinylpyrrolidone (PVP)), and glidants (such as silicon dioxide and magnesium stearate). In some embodiments, the diluent can be present in an amount from about 35% to about 90% by weight of the composition. In some embodiments, the glidant can be present in an amount from about 0.1% to about 5% by weight of the composition. In some embodiments, the disintegrant can be present in an amount from about 0.5% to about 10% by weight of the composition. In some embodiments, the granulation binders can be present in an amount from about 0.5% to about 5% by weight of the composition. In some embodiments of the second pharmaceutical composition, the at least one pharmaceutically acceptable excipient is chosen from mannitol, microcrystalline cellulose, sodium starch glycolate, polyvinylpyrrolidone (PVP), and magnesium stearate. In some embodiments of the second pharmaceutical composition, the at least one pharmaceutically acceptable excipient is chosen from microcrystalline cellulose, sodium starch glycolate, silicon dioxide, and magnesium stearate. In some embodiments of the second pharmaceutical composition, the at least one pharmaceutically acceptable excipient is chosen from microcrystalline cellulose, sodium starch glycolate, and magnesium stearate. In some embodiments of the second pharmaceutical composition, the at least one pharmaceutically acceptable excipient is chosen from microcrystalline cellulose and magnesium stearate.

In some embodiments of the second pharmaceutical composition, the pharmaceutical composition is in form of tablet or capsule, the micronized Compound A can be present in an amount of 1, 5,10, 15, 20, 25, 50, 75, 80, 85, 90, 95, 100, 125, 150, 200, 250, 300, 400 and 500 mg in a tablet or capsule, such as in a capsule.

Also disclosed herein is a third pharmaceutical composition comprising micronized Compound A that is Form I or substantially pure Form I , and at least one pharmaceutically acceptable excipient, wherein

the micronized Form I or substantially pure Form I has a D90 value of less than or equal to 20.0 μm, such as ranging from 1.0-20.0 μm, or ranging from 2.0-12.0 μm. In some embodiments of the third pharmaceutical composition, the micronized Form I or micronized substantially pure Form I has a D90 value ranging from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments of the third pharmaceutical

composition, the micronized Form I or micronized substantially pure Form I has a D90 value ranging from 2.0 to 5.0 μm, for example, has a D90 value as 3.0, 3.5, or 4.0 μm. In some embodiments of the third pharmaceutical composition, the micronized Form I or micronized substantially pure Form I has a D90 value ranging from 9.0 to 12.0 μm, for example, has a D90 value as 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

In some embodiments of the third pharmaceutical composition, the at least one pharmaceutically acceptable excipient is chosen from diluents (such as mannitol, microcrystalline cellulose, starch, lactose, dextrin, sorbitol), disintegrants (such as sodium starch glycolate), granulation binders (such as polyvinylpyrrolidone (PVP)), and glidants (such as silicon dioxide and magnesium stearate). In some embodiments, the diluent can be present in an amount from about 35% to about 90% by weight of the composition. In some embodiments, the glidant can be present in an amount from about 0.1% to about 5% by weight of the composition. In some embodiments, the disintegrant can be present in an amount from about 0.5% to about 10% by weight of the composition. In some embodiments, the granulation binders can be present in an amount from about 0.5% to about 5% by weight of the composition. In some embodiments of the third pharmaceutical composition, the at least one pharmaceutically acceptable excipient is chosen from mannitol, microcrystalline cellulose, sodium starch glycolate, polyvinylpyrrolidone (PVP), and magnesium stearate. In some embodiments of the third pharmaceutical composition, the at least one pharmaceutically acceptable excipient is chosen from microcrystalline cellulose, sodium starch glycolate, silicon dioxide, and magnesium stearate. In some embodiments of the third pharmaceutical composition, the at least one pharmaceutically acceptable excipient is chosen from microcrystalline cellulose, sodium starch glycolate, and magnesium stearate. In some embodiments of the third pharmaceutical composition, the at least one pharmaceutically acceptable excipient is chosen from microcrystalline cellulose and magnesium stearate.

In some embodiments of the third pharmaceutical composition, the pharmaceutical composition is in form of tablet or capsule, the micronized Compound A that is Form I or substantially pure Form I can be present in an amount of 1, 5,10, 15, 20, 25, 50, 75, 80, 85, 90, 95, 100, 125, 150, 200, 250, 300, 400 and 500 mg in a tablet or capsule, such as in a capsule.

Also disclosed herein is the compound and the composition as defined in the claims for use in a method of treating a subject in recognized need of treatment for at least one disease responsive to FGFR1 inhibitionand/or at least one disease responsive to KDR inhibition, wherein said disease is an angiogenesis-related disorders, of a pharmaceutical composition selected from the first, second, and third pharmaceutical composition, including each of the embodiments thereof, as disclosed above. In some embodiments, angiogenesis-related disorders as described herein are cancer and age-related macular degeneration. In some embodiments, cancers as described herein are lung cancer, head and neck cancer, colorectal cancer, pancreatic cancer, colon cancer, breast cancer, ovarian cancer, prostate cancer, stomach cancer, kidney cancer, liver cancer, brain cancer, bone cancer, thyroid cancer, neuroendocrine tumors, sarcoma, such as soft tissue sarcoma, and leukemia.

Also disclosed is a method of preparing a tablet or capsules, comprising:

mixing at least one active pharmaceutical ingredient chosen from Compound A, pharmaceutically acceptable salts thereof, and Form I, substantially pure Form I, Form II or substantially pure Form II of Compound A, with at least one pharmaceutically acceptable excipient, wherein Compound A, pharmaceutically acceptable salts thereof, and Form I, substantially pure Form I, Form II or substantially pure Form II of Compound A are micronized with PSD D90 of equal to or less than 20 um,
dry blending, wet granulating, or roller compacting the resulting mixture, and
filling into capsules the dry blended, wet granulated, or roller compacted mixture, or compressing into tablets the dry blended, wet granulated, or roller compacted mixture.

In some embodiments of the method of preparing a tablet or capsules, the Compound A, pharmaceutically acceptable salts thereof, and Form I, substantially pure Form I, Form II or substantially pure Form II of Compound A are micronized with PSD D90 value ranging from 1.0-20.0 μm, or ranging from 2.0-12.0 μm. In some embodiments of the method of preparing a tablet or capsules, the Compound A, pharmaceutically acceptable salts thereof, and Form I, substantially pure Form I, Form II or substantially pure Form II of Compound A are micronized with PSD D90 value ranging from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments of the method of preparing a tablet or capsules, the Compound A, pharmaceutically acceptable salts thereof, and Form I, substantially pure Form I, Form II or substantially pure Form II of Compound A are micronized with PSD D90 value ranging from 2.0 to 5.0 μm, for example, with a D90 value as 3.0, 3.5, or 4.0 μm. In some embodiments of the method of preparing a tablet or capsules, the Compound A, pharmaceutically acceptable salts thereof, and Form I, substantially pure Form I, Form II or substantially pure Form II of Compound A are micronized with PSD D90 value

ranging from 9.0 to 12.0 μm, for example, with a D90 value as 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

## BRIEF DESCRIPTION OF THE DRAWINGS

FIG. 1 shows anti-tumor effects of Form I of Compound A in NCI-H716 tumor model.

FIG. 2 shows PK profiles of Compound A in Beagle dogs of Group 1 after a single PO dosing of Capsule A at the dosage of 30 mg Form I of Compound A/kg body weight in Period 1.

FIG. 3 shows PK profiles of Compound A in Beagle dogs of Group 1 after a single PO dosing of Capsule B at the dosage of 30 mg Form I of Compound A/kg body weight in Period 2.

FIG. 4 shows PK profiles of Compound A in Beagle dogs of Group 2 after a single PO dosing of Capsule B at the dosage of 30 mg Form I of Compound A/kg body weight in Period 1.

FIG. 5 shows PK profiles of Compound A in Beagle dogs of Group 2 after a single PO dosing of Capsule A at the dosage of 30 mg Form I of Compound A/kg body weight in Period 2.

FIG. 6 shows Mean concentration-time profiles of Compound A in Beagle dog plasma after single PO dosing of Capsules A and B comprising different formulations (n=6, only the animals without emesis) are used to calculate the mean concentration).

FIG. 7 is X-ray powder diffractogram of Form I of Compound A and is the same as Figure 1 as disclosed in the '658 patent.

FIG. 8 is X-ray powder diffractogram of Form II of Compound A and is the same as Figure 5 as disclosed in the '658 patent.

The following abbreviations and terms have the indicated meanings throughout:

Unless clearly indicated otherwise, use of the terms "a", "an" and the like refers to one or more.

The term "D90 value" refers to 90% (by volume) of the particles have a size that is less than or equal to the value. For example, D90 value of 20.0 μm means 90% (by volume) of the particles is less than or equal to 20.0 μm in size; D90 value of 10.0 μm means 90% (by volume) of the particles is less than or equal to 10.0 μm in size.

The term "pharmaceutically acceptable" used herein refers to a substance which is, within the scope of sound medical judgment, suitable for use in contact with the body of human beings and other animals without excessive toxicity, irritation, allergic response, or other problem, commensurate with a reasonable benefit/risk ratio.

"Pharmaceutically acceptable salts" include, salts with inorganic acids, such as hydrochlorate, hydrobromate, phosphate, biphosphate, sulfate, sulfinate, nitrate,; as well as salts with an organic acid, such as malate, maleate, mandelate, fumarate, tartrate, succinate, citrate, aspartate, glutamate, atrolactate, gluconate, propionate, lactate, camphorsulfonate, methanesulfonate, ethanesulfonate, naphthalenesulfonate, p- toluenesulfonate, 2-hydroxyethylsulfonate, hydroxybutyrate, benzoate, salicylate, stearate, and alkanoate such as acetate, salt of HOOC-$(CH_2)$n-COOH where n is 0-4,. Where appropriate, "pharmaceutically acceptable salts" can also be base addition salts, and include sodium salt, potassium salt, calcium salt, aluminum salt, lithium salt, and ammonium salt.

In addition, if a compound described herein is obtained as an acid addition salt, the free base can be obtained by basifying a solution of the acid salt. Conversely, if the product is a free base, an acid addition salt, particularly a pharmaceutically acceptable acid addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used within the realm of routine experimentation to prepare non-toxic pharmaceutically acceptable addition salts.

The term "effective amount" used herein refers to an amount of Compound A or a pharmaceutically acceptable salt thereof, Form I, substantially pure Form I, Form II, or substantially pure Form II that, when administered to a subject, will elicit the biological or medical response of the subject, for example, ameliorate or eliminate one or more symptoms of a disease, alleviate or cure a disease, slow or delay progression of a disease etc.

The term "subject" used herein refers to an animal. Typically the animal is human beings or other animals, especially human beings and other mammals, for example, primates, cows, sheep, goats, horses, dogs, cats, rabbits. In some embodiments, the subject is a human.

The term "Form I" refers to Form I *N*-(2-(dimethylamino)ethyl)-1-(3-((4-((2-methyl-1*H*-indol-5-yl)oxy)pyrimidin-2-yl)amino)phenyl)-methanesulfonamide having peaks (2θ) chosen from those having the following values: 7.0, 8.0, and 8.6, each of the diffraction angles being ± 0.2 degrees (2θ). In some embodiments, the X-ray powder diffractogram of the Form I as described herein may have peaks (2θ) chosen from those having the following values: 7.0, 8.0, 8.6, 11.0, 11.8 , each of the diffraction angles being ± 0.2 degrees (2θ). In some embodiments, the Form I as described herein

have a X-ray powder diffractogram as shown in Figure 7.

The term "Form II" refers to Form II *N*-(2-(dimethylamino) ethyl)-1-(3-((4-((2-methyl-1*H*-indol-5-yl)oxy)pyrimidin-2-yl)amino)phenyl)-methanesulfonamide having peaks (2θ) chosen from those having the following values: 6.8, 9.8, 10.5, and 10.7, each of the diffraction angles being ± 0.2 degrees (2θ). In some embodiments, the X-ray powder diffractogram of the Form II as described herein may have peaks (2θ) chosen from those having the following values: 6.8, 9.8, 10.5, 10.7, 13.6, 15.0, each of the diffraction angles being ± 0.2 degrees (2θ). In some embodiments, the Form II as described herein may have a X-ray powder diffractogram as shown in Figure 8.

The term "substantially pure Form I" refers to Compound A wherein at least 75% by weight of Compound A is Form I. For example, "substantially pure Form I" refers to Compound A wherein at least 75%, 80%, 85%, 90%, 95%, or 100% by weight of Compound A is Form I.

The term "substantially pure Form II" refers to Compound A wherein at least 75% by weight of Compound A is Form II. For example, "substantially pure Form II" refers to Compound A wherein at least 75%, 80%, 85%, 90%, 95%, or 100% by weight of Compound A is Form II.

The word "about" in conjunction with a value extends it to a range of ±20% of said value. For example, about 5% means a range of 4% to 6%.

In some embodiments, at least one active pharmaceutical ingredient chosen from the micronized compound of Formula A (Compound A) and/or micronized pharmaceutically acceptable salts thereof, and micronized Form I, micronized substantively pure Form I, Form II, micronized substantively pure Form II of the compound of Formula A having a particle size distribution (PSD) D90 value of less than or equal to 20 μm may be useful for the treatment of neuroendocrine tumors.

In some embodiments, it is disclosed micronized Form I *N*-(2-(dimethylamino)ethyl)-1-(3-((4-((2-methyl-1*H*-indol-5-yl)oxy)pyrimidin-2-yl)amino)phenyl)methanesulfonamide having a particle size distribution (PSD) D90 value of less than or equal to 20 μm for use in the treatment of said neuroendocrine tumors.

In some embodiments, it is disclosed micronized Form II *N*-(2-(dimethylamino) ethyl)-1-(3-((4-((2-methyl-1*H*-indol-5-yl)oxy)pyrimidin-2-yl)amino)phenyl)methanesulfonamide having a particle size distribution (PSD) D90 value of less than or equal to 20 μm for use in the treatment of said neuroendocrine tumors.

In some embodiments, a pharmaceutical composition comprising: at least one pharmaceutically acceptable excipient and the micronized compound of Formula A and/or a micronized pharmaceutically acceptable salts thereof, having a particle size distribution (PSD) D90 value of less than or equal to 20 μm, for use in said treatment.

In some embodiments, it is disclosed a pharmaceutical composition comprising: at least one pharmaceutically acceptable excipient and micronized Form I *N*-(2-(dimethylamino)ethyl)-1-(3-((4-((2-methyt-1*H*-indol-5-yl)oxy)pyrimidin-2-yl)amino)phenyl)methanesulfonamide having a particle size distribution (PSD) D90 value of less than or equal to 20 μm for use in said treatment.

In some embodiments, it is disclosed a pharmaceutical composition comprising: at least one pharmaceutically acceptable excipient and micronized Form II *N*-(2-(dimethytamino)ethyl)-1-(3-((4-((2-methyt-1*H*-indot-5-yl)oxy)pyrimidin-2-yl)amino)phenyl)methanesulfonamide having a particle size distribution (PSD) D90 value of less than or equal to 20 μm for use in said treatment.

In all embodiments disclosed herein above and hereinafter, Compound A, at least one pharmaceutically acceptable salt thereof, Form I, and Form **II** *N*-(2-(dimethylamino)ethyl)-1-(3-((4-((2-methyl-1*H*-indol-5-yl)oxy)pyrimidin-2-yl)amino)phenyl)methanesulfonamide, and substantially pure Form I and Form II *N*-(2-(dimethylamino)ethyl)-1-(3-((4-((2-methyl-1*H*-indol-5-yl)oxy)pyrimidin-2-yl)amino)phenyl)methanesulfonamide are micronized, with a D90 value of less than or equal to 20.0 μm, such as ranging from 1.0- 20.0 μm, or ranging from 2.0-12.0 μm, further such as from 1.0 to 2.0 μm, from greater than 2.0 to 3.0 μm, from greater than 3.0 to 4.0 μm, from greater than 4.0 to 6.0 μm, from greater than 6.0 to 8.0 μm, from greater than 8.0 to 10.0 μm, or from greater than 10.0 to 12.0 μm. In some embodiments, the D90 value ranges from 2.0 to 5.0 μm, for example, the D90 value is 3.0, 3.5, or 4.0 μm. In some embodiments, the D90 value ranges from 9.0 to 12.0 μm, for example, the D90 value is 9.5 or 10.0 μm. In a preferred embodiment, the D90 value is less than or equal to 11.0 μm or is less than or equal to 10.0 μm. In a more preferred embodiment, the D90 value is less than or equal to 6.0 μm. In a particular embodiment, the D90 value is less than or equal to 4.0 μm.

The amount of the at least one active pharmaceutical ingredient chosen from the compound of Formula A and/or pharmaceutically acceptable salts thereof and Form I, substantively pure Form I, Form II, substantively pure Form II of the compound of Formula A effective for achieving the desired biological effect may depend on a number of factors, for example, the intended use, the mode of administration, and the clinical condition of the patient. The daily dose may, for example, range from 0.1 mg to 3 g/day (such as from 0.5 mg to 2 g /day, further such as from 50 mg to 1g /day). Single-dose formulations which can be administered orally include, for example, tablets or capsules. Further for example, the at least one active pharmaceutical ingredient chosen from the compound of Formula A and/or pharmaceutically acceptable salts thereof and Form I, substantively pure Form I, Form II, substantively pure Form II of the compound of Formula A can be present in an amount of 1, 5, 10, 15, 20, 25, 50, 75, 80, 85, 90, 95, 100, 125, 150, 200, 250, 300, 400 and 500 mg in a capsule or tablet.

For the therapy of the above-mentioned conditions, the at least one active pharmaceutical ingredient chosen from the micronized compound of Formula A and/or micronized pharmaceutically acceptable salts thereof and micronized Form I , substantively pure Form I, Form II, substantively pure Form II of the compound of Formula A having a particle size distribution (PSD) D90 value of less than or equal to 20 $\mu$m may be used as the compound itself, but typically each of them would be used in the form of a pharmaceutical composition with one or more pharmaceutically acceptable excipients. Representative excipients should be compatible with the other ingredients of the composition and not harmful for the patient's health. The excipient may be a solid or a liquid or both and may be formulated with the compound of Formula A, such as Form I and/or Form II described herein, as a single dose, for example as a tablet or a capsule, which may be prepared from 0.05% to 95% by weight of the compound of Formula A described herein. The pharmaceutical compositions described herein can be produced by known pharmaceutical methods, such as those involving mixing the at least one active pharmaceutical ingredient chosen from the compound of Formula A and/or pharmaceutically acceptable salts thereof and Forms I and II of the compound of Formula A with pharmaceutically acceptable excipients.

In some embodiments, representative excipients would include: diluents (such as cellulose, starch, lactose, mannitol, dextrin, sorbitol, etc.), surfactants (such as sodium lauryl sulfate, poloxamer, etc.), solubilizers (such as polyvinylpyrrolidone, polyethylene glycol, etc.), disintegrants (such as sodium starch glycolate, PVPP, Croscarmellose, etc.) and glidant and lubricant (such as $SiO_2$, magnesium stearate, etc.). Further exemplary excipients include microcrystalline cellulose, sodium citrate, calcium carbonate, dicalcium phosphate, glycine, disintegrants such as starch, sodium cross-linked carboxymethyl cellulose, composite silicates, and polyethylene glycol with high moleculr weight, granulation binders (such as polyvinylpyrrolidone, sucrose, gelatin, and Gum Arabic), and lubricants (such as stearic acid, sodium stearyl fumarate and talc). Further exemplary excipients include diluents (such as mannitol and microcrystalline Cellulose) and glidants (such as magnesium stearate or silicon dioxide). Further exemplary excipients include diluents (such as mannitol and microcrystalline Cellulose), glidants (such as magnesium stearate or silicon dioxide), and disintegrants (such as sodium starch glycolate). Further exemplary excipients include diluents (such as mannitol and microcrystalline Cellulose), glidants (such as magnesium stearate or silicon dioxide), disintegrants (such as sodium starch glycolate), and granulation binders (such as polyvinylpyrrolidone).

In some embodiments, the at least one active pharmaceutical ingredient chosen from the compound of Formula A and/or pharmaceutically acceptable salts thereof and Form I , substantively pure Form I, Form II, substantively pure Form II of the compound of Formula A may be combined with at least one component, such as excipient chosen from sweeteners, delicate flavor agents, coloring matters, dyes, and emulsifiers.

In some embodiments, the Form I or Form II described herein may not be converted upon formulation with the one or more pharmaceutically acceptable diluents. In other embodiments, the Form I or Form II described herein may be converted, in whole or in part, to one or more other forms, including a non-solid form, upon formulation with the one or more pharmaceutically acceptable diluents. Exemplary diluents would include water, ethanol, propylene glycol, glycerine, and mixtures thereof. In some embodiments, the Form I or Form II described herein can be dissolved when formulated into a pharmaceutical composition. Accordingly, in such "dissolved" cases, the Form I or Form II no longer exists in their respective crystalline forms in the pharmaceutical composition and is equivalent to any form of Compound A as dissolved.

In some embodiments, the at least one active pharmaceutical ingredient chosen from the compound of Formula A and/or pharmaceutically acceptable salts thereof and Form I , substantively pure Form I, Form II, substantively pure Form II of the compound of Formula A may be formulated to a suitable form.

Pharmaceutical compositions described herein can be those suitable for oral and peroral (for example sublingual) administration, although the suitable mode of administration may depend in each individual case on the nature and severity of the condition to be treated and on the nature of the at least one active pharmaceutical ingredient chosen from the compound of Formula A and/or pharmaceutically acceptable salts thereof and Form I, substantively pure Form I, Form II, substantively pure Form II of the compound of Formula A used in each case to prepare the pharmaceutical composition. Coated formulations and coated slow-release formulations also are provided. Acid- and gastric juice-resistant formulations are possible. Suitable coatings resistant to gastric juice comprise cellulose acetate phthalate, polyvinyl acetate phthalate, hydroxypropylmethylcellulose phthalate, anionic polymers of methacrylic acid, and methyl methacrylate.

Suitable pharmaceutical compositions for oral administration prepared from the at least one active pharmaceutical ingredient chosen from the compound of Formula A and/or pharmaceutically acceptable salts thereof and Form I , substantively pure Form I, Form II, substantively pure Form II of the compound of Formula A may be in the form of separate units such as, for example, capsules, cachets, and tablets, including suckable tablets, each of which may be prepared with a defined amount of the at least one active pharmaceutical ingredient described herein; as well as in the forms chosen from powders, granules, solutions, suspensions in an aqueous or nonaqueous liquid, and oil-in-water and water-in-oil emulsions. Those compositions may, as already mentioned, be prepared by any suitable pharmaceutical formulation method, such as those including a step wherein the at least one active pharmaceutical ingredient chosen from the compound of Formula A and/or pharmaceutically acceptable salts thereof and Form I , substantively pure Form

I, Form II, substantively pure Form II of the compound of Formula A and a excipient (which may consist of one or more additional ingredients, including diluents) are brought into contact. The compositions can generally be produced by uniform and homogeneous mixing of the at least one active pharmaceutical ingredient chosen from the compound of Formula A and/or pharmaceutically acceptable salts thereof and Form I , substantively pure Form I, Form II, substantively pure Form II of the compound of Formula A with a liquid and/or finely divided solid excipient, after which the product can be shaped. Thus, for example, a tablet can be produced by compressing or molding a powder or granules of the at least one active pharmaceutical ingredient chosen from the compound of Formula A and/or pharmaceutically acceptable salts thereof and Form I, substantively pure Form I, Form II, substantively pure Form II of the compound of Formula A, where appropriate with one or more additional ingredients. Compressed tablets can be produced by tableting the at least one active pharmaceutical ingredient chosen from the compound of Formula A and/or pharmaceutically acceptable salts thereof and Form I , substantively pure Form I, Form II, substantively pure Form II of the compound of Formula A in free-flowing form such as, for example, a powder or granules, where appropriate mixed with a binder, glidant, inert diluent and/or one (or more) surface-active/dispersing agent(s) in a suitable machine. Molded tablets can be produced by molding the at least one active pharmaceutical ingredient chosen from the compound of Formula A and/or pharmaceutically acceptable salts thereof and Form I, substantively pure Form I, Form II, substantively pure Form II of the compound of Formula A in powder form and then moistening with an inert liquid diluent, in a suitable machine. Compositions can also be prepared by wet granulation. Thus, for example, a composition can be prepared by wet granulation by mixing the at least one active pharmaceutical ingredient chosen from the compound of Formula A and/or pharmaceutically acceptable salts thereof and Form I, substantively pure Form I, Form II, substantively pure Form II of the compound of Formula A with one or more optional additional ingredients, a suitable solvent, and a binder to prepare a wet granulate, drying the wet granulate, and milling the dried granulate. The method may further comprise adding at least one lubricant to the dried milled granulate and compressing the dried milled granulate to form tablets. The optional additional ingredients may include, for example, at least one diluent and/or at least one disintegration agent. The suitable solvent can be water. In some embodiments, the diluent is chosen from calcium carbonate, calcium phosphate (dibasic and/or tribasic), calcium sulfate, powdered cellulose, dextrates, dextrin, fructose, kaolin, lactitol, anhydrous lactose, lactose monohydrate, maltose, mannitol, microcrystalline cellulose, sorbitol, sucrose, and starch. In some embodiments, the diluent can be present in an amount from about 35% to about 90% by weight of the tablet. In some embodiments, the binder can be chosen from acacia, alginic acid, carbomer, sodium carboxymethylcellulose, dextrin, ethylcellulose, gelatin, glucose, guar gum, hydroxypropyl cellulose, maltose, methylcellulose, polyethylene oxide, and povidone. In some exemplary embodiments, the binder is present in an amount of about 0.5% to about 5% by weight of the tablet. In other exemplary embodiments, the above-mentioned preparations contain about 0.05-5 g of the at least one active pharmaceutical ingredient chosen from the compound of Formula A and/or pharmaceutically acceptable salts thereof and Form I , substantively pure Form I, Form II, substantively pure Form II of the compound of Formula A per milliliter or per gram of the preparations.

The compositions disclosed herein can be administered topically or systemically.

Pharmaceutical compositions which are suitable for peroral (sublingual) administration can comprise suckable tablets which can be prepared from the at least one active pharmaceutical ingredient chosen from the compound of Formula A and/or pharmaceutically acceptable salts thereof and Form I , substantively pure Form I, Form II, substantively pure Form II of the compound of Formula A, with a flavoring agent, normally chosen from sucrose, gum arabic, tragacanth, and pastilles.

Pharmaceutical compositions described herein can also be those suitable for parenterally administration, by inhalation spray, or via an implanted reservoir. Solid excipients, for example, starch, lactose, Microcrystalline Cellulose, aluminum silicate, liquid excipients, for example, injectable water, polyvinyl alcohol, non-ionized surfactant agents, and corn oil, and any ingredients suitable for intend use. Other excipients commonly used in pharmaceutical formulation include coloring agents, preservatives, taste correctives agents and antioxidants such as vitamin E, vitamin A, BHT and BHA.

The compound of Formula A, such as the Form I or Form II described herein, can also be administrated intraperitoneally. And the solution and suspension of those compounds can be prepared by dissolving or suspended the compound in water containing suitable surfactants. Dispersed suspensions can be prepared by using glycerol, polyethylene glycol (PEG) or their mixture with suitable oils. Preservatives agents can be added to those formulations to prevent growth of microorganisms during use.

Injectable formulations include solution or suspension in sterilized water, and sterilized powder. In all cases, those formulations must be sterilized and easily removed from the syringe, and stable under the manufacture and storage conditions, and as free as possible from pollution and the effects of microorganisms. Excipients can be solvents or dispersing agents, and include water, alcohol, and some suitable oils.

The at least one active pharmaceutical ingredient chosen from the compound of Formula A and/or pharmaceutically acceptable salts thereof and Form I , substantively pure Form I, Form II, substantively pure Form II of the compound of Formula A can also be administered in combination with one or more other active ingredients. When administered as a combination, the active ingredients can be formulated as separate compositions that are administered

at the same time or sequentially at different times, or the active ingredients can be administered in a single dosage form, i.e., single composition, provided that the active ingredients are not, in that single dosage form, incompatible with other active ingredients or the formulation, or otherwise undesirably combined in a single composition.

In some embodiments, the at least one active pharmaceutical ingredient chosen from the compound of Formula A and/or pharmaceutically acceptable salts thereof and Form I , substantively pure Form I, Form II, substantively pure Form II of the compound of Formula A can be administered with one or more other agents known for the treatment of at least one disease responsive to FGFR1 inhibition, such as cancer, and/or at least one disease responsive to KDR inhibition, such as angiogenesis-related disorders.

The phrase "co-therapy" (or "combination-therapy") or "in combination with", as used herein, defines the use of the at least one active pharmaceutical ingredient chosen from the compound of Formula A and/or pharmaceutically acceptable salts thereof and Form I, substantively pure Form I, Form II, substantively pure Form II of the compound of Formula A as described herein and one or more other active ingredients, such as, for example, anti-neoplastic agents. As used herein, the term "anti-neoplastic agent" refers to any agent or treatment that is administered to a subject with cancer for purposes of treating the cancer, including: radiotherapy; immunotherapy; DNA damaging chemotherapeutic agents; and chemotherapeutic agents that disrupt cell replication.

Non-limiting examples of DNA damaging chemotherapeutic agents include topoisomerase I inhibitors (e.g., irinotecan, topotecan, camptothecin and analogs or metabolites thereof, and doxorubicin); topoisomerase II inhibitors (e.g., etoposide, teniposide, and daunorubicin); alkylating agents (e.g., melphalan, chlorambucil, busulfan, thiotepa, ifosfamide, carmustine, lomustine, semustine, streptozocin, dacarbazine, methotrexate, mitomycin C, and cyclophosphamide); DNA intercalators (e.g., cisplatin, oxaliplatin, and carboplatin); DNA intercalators and free radical generators such as bleomycin; and nucleoside mimetics (e.g., 5-fluorouracil, capecitabine, gemcitabine, fludarabine, cytarabine, mercaptopurine, thioguanine, pentostatin, and hydroxyurea).

Chemotherapeutic agents that disrupt cell replication include: paclitaxel, docetaxel, and related analogs; vincristine, vinblastin, and related analogs; thalidomide and related analogs (e.g., CC-5013 and CC-4047); protein tyrosine kinase inhibitors (e.g., imatinib mesylate and gefitinib); proteasome inhibitors (e.g., bortezomib); NF-kappa B inhibitors, including inhibitors of I kappa B kinase; antibodies which bind to proteins overexpressed in cancers and thereby downregulate cell replication (e.g., trastuzumab, rituximab, cetuximab, and bevacizumab); and other inhibitors of proteins or enzymes known to be upregulated, over-expressed, or activated in cancers, the inhibition of which downregulates cell replication.

In co-therapy, administration of each active ingredient can occur in a sequential manner in a regimen to provide beneficial effects of the drug combination; and/or co-administration of the aforementioned components can occur in a substantially simultaneous manner (e.g., as in a single dosage form, such as a capsule, having a fixed ratio of the active ingredients or in multiple, separate capsules for each active ingredient, etc.).

Thus, methods described herein are not limited in the sequence of administration; the at least one active pharmaceutical ingredient chosen from the compound of Formula A and/or pharmaceutically acceptable salts thereof and Form I , substantively pure Form I, Form II, substantively pure Form II of the compound of Formula A described herein may be administered either prior to, at the same time with or after administration of the one or more other active ingredients.

The following non-limiting examples are provided.

**Example 1: Formulations**

Unless otherwise indicated, D90 values were obtained through laser particle size distribution analysis method using Malvern 3000 and HYDRO MV or equivalent with particle refractive index at 1.59, absorbable index at 0.01, and stirrer speed at 2500rpm, and without ultrasonic. Sample preparation for wet measurement comprised: placing approximately 30mg of sample into a 20ml glass bottle; adding 20 drops 0.25% Triton X-100 (w/v)-water to wet sample, and then adding 10ml purified water, sonicating for 2 minute. The sample was analyzed within 2 minutes.

Compound A used in the following examples is Form I .

The capsules and tablets described in the Examples were analyzed for the release rate of Compound A into 900 ml aqueous media (USP pH 4.5 acetate buffer) at 75 rpm rotation according to the first method (basket) described in the China Pharmacopoeia (2010 Edition).

Formulation process 1-Wet granulation

A. 50mg capsule, 200mg capsule

The 200 mg capsule formulation (200 mg Capsule No. 1), in which the components are as shown in Table 1, was prepared from 500 gram of the micronized Compound A (PSD, D90= 9.6μm), 277.5 grams of mannitol, 150 grams

of microcrystalline cellulose, 50 grams of sodium starch glycolate, 12.5 grams of polyvinylpyrrolidone (PVP) K30, and 10 grams of magnesium stearate. The micronized Compound A (500 g, D90=9.6 μm), mannitol (277.5 g), microcrystalline cellulose(150 g), and sodium starch glycolate (50 g) were mixed together. PVP-K30 (12.5 g) in a 5% (W/V) aqueous solution was prepared and added as binder to prepare granules by wet granulation process. Magnesium stearate (10 g) were added and blended for 3 minutes. The final blend containing 200 mg of Compound A was filled into each size #0 capsule to prepare Compound A 200 mg capsule products(200 mg Capsule No. 1). The accumulative release of Compound A from the 200mg capsule was 86.7% at 30min. The stability tests indicated that this capsule sample was stable at 25°C/60%RH for 12 months and at 40°C/75%RH for 6 months, without any content assay changed and degradation detected. The stability test is on-going.

Capsules of other dosages were prepared similarly. For example, 50 mg capsule of the micronized Compound A (D90=9.6μm) (50 mg Capsule No. 1) was prepared by filling a final blend containing 50 mg of Compound A prepared in a manner similar to the above into each size #3 capsule, wherein the components are as shown in Table 1. The accumulative release of Compound A from the 50mg capsule was 90.8% at 30min. The stability tests indicated that this capsule sample was stable at 25°C/60%RH for 12 months and at 40°C/75%RH for 6 months, without any content assay changed and degradation detected.

Table 1. The formulation examples by wet granulation process

| Components | 200mg capsule No. 1 | 50mg capsule No. 1 |
|---|---|---|
| Compound A | 200mg | 50mg |
| Mannitol | 111mg | 27.75mg |
| Microcrystalline Cellulose | 60mg | 15mg |
| Sodium Starch Glycolate | 20mg | 5mg |
| Povidone (PVP) | 5mg | 1.25mg |
| Magnesium stearate | 4mg | 1mg |

Another 50 mg capsule formulation (50 mg Capsule No. 2), in which the components are as shown in Table 2, was prepared from 15 gram of the micronized Compound A (PSD, D90=3.8μm),19.9875 grams of mannitol, 6.75 grams of microcrystalline cellulose, 2.25 grams of sodium starch glycolate, 0.5625 grams of polyvinylpyrrolidone (PVP) K30, and 0.45 grams of magnesium stearate. The micronized Compound A (Form I, 15 g, D90=3.8 μm), mannitol (19.9875 g), microcrystalline cellulose (6.75 g), and sodium starch glycolate (2.25 g) were mixed together. PVP-K30 (0.5625 g) in a 3% (W/V) aqueous solution was prepared and added as binder to prepare granules by wet granulation process. After the resulting wet granules were dried, magnesium stearate (0.45 g) was added to the dried granules and then blended for 3 minutes. The final blend containing 50 mg of Compound A was filled into each size #3 capsule to prepare Compound A 50 mg capsule products (50 mg Capsule No. 2). The accumulative release of Compound A from the 50mg capsule was 89.2% at 30min.

Another 50mg capsule formulation (50 mg Capsule No. 3), in which the components are as shown in Table 2, was prepared from 5 gram of the micronized Compound A (PSD, D90=3.8μm),15.37 grams of mannitol, 5.6 grams of microcrystalline cellulose, 1.4 grams of sodium starch glycolate, 0.35 grams of polyvinylpyrrolidone (PVP) K30, and 0.28 grams of magnesium stearate. The micronized Compound A (5 g, D90=3.8 μm), mannitol (15.37 g), microcrystalline cellulose (5.6 g), and sodium starch glycolate (1.4 g) were mixed together. PVP-K30 (0.35 g) in a 3% (W/V) aqueous solution was prepared and added as binder to prepare granules by wet granulation process. After the wet granules were dried, magnesium stearate (0.28g) was added to the dried granules and then blended for 3 minutes. The final blend containing 50 mg of Compound A was filled into each size #1 capsule to prepare Compound A 50 mg capsule products (50 mg Capsule No. 3). The accumulative release of Compound A from the 50mg capsule was 86.5% at 30min.

Another 50mg capsule formulation (50 mg Capsule No. 4), in which the components are as shown in Table 2, was prepared from 120 gram of the micronized Compound A (PSD, D90=5.7μm),73.92 grams of mannitol, 135.48 grams of microcrystalline cellulose, 18 grams of sodium starch glycolate, 9 grams of polyvinylpyrrolidone (PVP) K30, and 3.6 grams of magnesium stearate. The micronized Compound A (120 g), mannitol (73.92 g), microcrystalline cellulose(135.48 g), and sodium starch glycolate (18g) were mixed together. PVP-K30 (9 g) in a 10% (W/V) aqueous solution was prepared and added as binder to prepare granules by wet granulation process. After the wet granules were dried, magnesium stearate was added to the dried granules and then blended for 3 minutes. The final blend containing 50 mg of Compound A was filled into each size #3 capsule to prepare Compound A 50 mg capsule products (50 mg Capsule No. 4). The accumulative release of Compound A from the 50mg capsule was 89.2% at 30min.

Table 2. The formulation examples by wet granulation process

| Components | 50mg capsule No. 2 | 50mg capsule No. 3 | 50mg capsule No. 4 |
|---|---|---|---|
| Compound A | 50mg | 50mg | 50mg |
| Mannitol | 66.625mg | 153.7mg | 30.8mg |
| Microcrystalline Cellulose | 22.5mg | 56mg | 56.45mg |
| Sodium Starch Glycolate | 7.5mg | 14mg | 7.5mg |
| Povidone (PVP) | 1.875mg | 3.5mg | 3.75mg |
| Magnesium stearate | 1.5mg | 2.8mg | 1.5mg |

B. 300mg tablet, 250mg tablet, 200mg tablet

The 300mg tablet formulation, in which the components are as shown in Table 3, was prepared from 4200 gram of the micronized Compound A (PSD, D90=4.8μm), 3360 grams of mannitol, 1512 grams of microcrystalline cellulose, 504 grams of sodium starch glycolate, 403.2 grams of Povidone (PVP) K30, and 100.8 grams of magnesium stearate. The micronized Compound A, mannitol, microcrystalline cellulose and sodium starch glycolate were mixed together. PVP-K30 in a 12% (W/V) aqueous solution was prepared and added as binder to prepare granules by wet granulation process. After the wet granules were dried, magnesium stearate was added to the dried granules and then blended for 3 minutes. The final blend was compressed into an oblong tablet with compression force of 184 kg for the 300 mg tablet, of 164 kg for the 250 mg tablet, and of 116 kg for the 200 mg tablet, which contains 300 mg of Compound A/tablet. The tablets were film-coated by spraying of Opadry® II. The accumulative release of Compound A from the 300mg tablet was 90.3% at 30min.

Tablets of other dosages (the 250mg tablet and 200mg tablet, in which the components are as shown in Table 3) were prepared similarly.

Table 3. The tablet formulation examples by wet granulation process

| Components | 200mg Tablet | 250 mg Tablet | 300 mg Tablet |
|---|---|---|---|
| Compound A | 200 mg | 250 mg | 300 mg |
| Microcrystalline cellulose | 72 mg | 90 mg | 108 mg |
| Mannitol | 160 mg | 200 mg | 240 mg |
| Sodium Starch Glycolate | 24 mg | 30 mg | 36 mg |
| Povidone (K30) | 19.2 mg | 24 mg | 28.8 mg |
| Magnesium Stearate | 4.8 mg | 6 mg | 7.2 mg |
| Opadry® II | 9.6 mg | 12 mg | 14.4 mg |
| Total (mg) | 489.6 mg | 612 mg | 734.4 mg |

Formulation process 2 - Direct mixing

A. 50 mg Capsule

Micronized Form I of Compound A (150 g, D90=10.0μm) and microcrystalline cellulose (238.05 g) were sifted and mixed homogenously. Then magnesium stearate (1.95 g) was added and blended. The final blend containing 50 mg of Compound A was filled into each size #3 capsule to prepare 50 mg Compound A capsule products (50 mg Capsule No. 5) with components shown in Table 4. The accumulative release of Compound A from the 50mg capsule was 96.5% at 30min.

Alternatively, micronized Form I of Compound A (25 g, D90=3.5 μm) and microcrystalline cellulose (39.675 g) were sifted and mixed homogenously. Then magnesium stearate (0.325 g) was added and blended. The final blend containing 50 mg of Compound A was filled into each size #3 capsule to prepare 50 mg Compound A capsule products (50 mg Capsule No. 6) with components shown in Table 4. The accumulative release of Compound A from the 50mg capsule was 95.3% at 30min. The stability tests indicated that these capsule samples were stable at 25°C/60%RH for

12 months and at 40°C/75%RH for 6 months, without any content assay changed and degradation detected.

Further alternatively, micronized Form I of Compound A (25 g, D90=5.2 μm) and microcrystalline cellulose (39.675 g) were sifted and mixed homogenously. Then magnesium stearate (0.325 g) was added and blended. The final blend containing 50 mg of Compound A was filled into each size #3 capsule to prepare 50 mg Compound A capsule products (50 mg Capsule No. 7) with components shown in Table 4. The accumulative release of Compound A from the 50mg capsule was 83.8% at 30min.

Further alternatively, micronized Form I of Compound A (10 g, D90=8.1μm) and microcrystalline cellulose (15.87 g) were sifted and mixed homogenously. Then magnesium stearate (0.13 g) was added and blended. The final blend containing 50 mg of Compound A was filled into each size #3 capsule to prepare 50 mg Compound A capsule products (50 mg Capsule No. 8) with components shown in Table 4. The accumulative release of Compound A from the 50mg capsule was 98.1% at 30min.

Further alternatively, micronized Form I of Compound A (5.0 g, D90=2.1μm) and microcrystalline cellulose (7.935 g) were sifted and mixed homogenously. Then magnesium stearate (0.065 g) was added and blended. The final blend containing 50 mg of Compound A was filled into each size #3 capsule to prepare 50 mg Compound A capsule products (50 mg Capsule No. 9) with components shown in Table 4. The accumulative release of Compound A from the 50mg capsule was 92.8% at 30min.

Further alternatively, micronized Form I of Compound A (10 g, D90=3.4μm), microcrystalline cellulose (5.1g), mannitol (16.69g),sodium starch glycolate (1.7 g), and silicon dioxide (0.17 g) were sifted and mixed homogenously. Then magnesium stearate (0.34g) was added and blended for 3 minutes. 50 mg of the micronized Compound A capsules (50 mg Capsule No. 10) with components shown in Table 4 was obtained by filling the final blend containing 50 mg of Compound A into each size #3 capsule. The accumulative release of Compound A from the 50mg capsule was 90.7% at 30min.

Table 4. The formulation examples by direct mixing and filling process

| | 50mg capsule No. 5 | 50mg capsule No. 6 | 50mg capsule No. 7 | 50mg capsule No. 8 | 50mg capsule No. 9 | 50mg capsule No. 10 |
|---|---|---|---|---|---|---|
| Compound A, Form I | 50 mg | 50 mg | 50 mg | 50 mg | 50 mg | 50 mg |
| Microcrystalline Cellulose | 79.35mg | 79.35mg | 79.35mg | 79.35mg | 79.35mg | 25.5 mg |
| Mannitol | - | - | - | - | - | 83.45mg |
| Sodium Starch Glycolate | - | - | - | - | - | 8.5 mg |
| Silicon dioxide | - | - | - | - | - | 0.85 mg |
| Magnesium stearate | 0.65 mg | 0.65 mg | 0.65 mg | 0.65 mg | 0.65 mg | 1.7 mg |

B. 200 mg capsule

Micronized Form I of Compound A (200 g, D90=10.0 μm), microcrystalline cellulose (178 g), sodium starch glycolate (9 g), and silicon dioxide (9 g) were sifted and mixed homogenously. Then magnesium stearate (4 g) was added and blended for 3 minutes. 200 mg of the micronized Compound A capsule (200 mg Capsule No. 2) was obtained by filling the final blend containing 200 mg of Compound A into each size #0 capsule. The accumulative release of Compound A from the 200mg capsule was 75.1% at 30min. The stability tests indicated that this capsule sample was stable at 25°C/60%RH for 12 months and at 40°C/75%RH for 6 months, without any content assay changed and degradation detected.

Alternatively, micronized Form I of Compound A (1050g, D90=10.0μm), microcrystalline cellulose (726.6g), sodium starch glycolate (75.6g) were sifted and mixed homogenously. Then magnesium stearate (37.8g) was added and blended for 3 minutes. 200 mg of the micronized Compound A capsule (200 mg Capsule No. 3) was obtained by filling the final blend containing 200 mg of Compound A into each size #0 capsule. The accumulative release of Compound A from the 200mg capsule was 84.6% at 30min. The stability tests indicated that this capsule sample was stable at 25°C/60%RH for 3 months and at 40°C/75%RH for 3 months, without any content assay changed and degradation detected.

Table 5. The formulation examples by direct mixing and filling process

| | 200mg capsule No. 2 | 200mg capsule No. 3 |
|---|---|---|
| Compound A, Form I | 200mg | 200mg |
| Microcrystalline Cellulose | 178mg | 138.4mg |
| Mannitol | - | - |
| Sodium Starch Glycolate | 9mg | 14.4mg |
| Silicon dioxide | 9mg | - |
| Magnesium stearate | 4mg | 7.2mg |

C. 25 mg capsule

Micronized Form I of Compound A (27.8 g, D90=3.3 μm) and microcrystalline cellulose (205.1 g) were sifted and mixed homogenously. Then magnesium stearate (0.67 g) was added and blended for 5 minutes. The final blend containing 25 mg of Compound A was filled into each size #1 capsule to prepare 25 mg Compound A capsule products. The accumulative release of Compound A from the 25mg capsule was 100% at 30min.

Table 6. The formulation example by direct mixing and filling process

| | 25mg capsule |
|---|---|
| Compound A, Form I | 25mg |
| Microcrystalline Cellulose | 184.4mg |
| Magnesium stearate | 0.6mg |

**Example 2: Anti-tumor effect of Compound A in H716 xenograft model**

Human colorectal adenocarcinoma cell line NCI-H716 cell line was reported to have the characteristic of endocrine cells, such as Dopa decarboxylase activity and cytoplasmic dense core granules. To confirm neuroendocrine characteristics of this cell line, subcutaneous tumors of NCI-H716 were established in nude mice, and pathological diagnosis with hematoxylin and eosin (HE) staining and immunohistochemistry (IHC) staining were performed on tumor tissue from subcutaneous xenograft model. NCI-H716 cells showed a diffuse growth pattern morphologically. The neoplastic cells were uniform and had large nuclei frequently with prominent nucleoli. IHC staining displayed that the tumor tissue was positive for CgA(chromogranin A), Syn(synaptophysin) and CD56(neural cell adhesion molecule 1), which was an important standard for neuroendocrine tumor diagnosis. Ki-67 index was around 80%. Based on the morphologic and immunohistochemistry findings, NCI-H716 xenograft could be considered as a neuroendocrine carcinoma.

**Materials and methods**

**Human tumor** cell line: NCI-H716 cell line was purchased from ATCC (CCL-251™) and incubated in the medium of RPMI1640 (Rosewell Park Memorial Institute 1640) plus 10% fetal bovine serum (FBS) at 37°C in a 5% $CO_2$ incubator.

**Animals:** BALB/c athymic male mice (6-8 weeks) were purchased from Shanghai SLAC Laboratory Animal Co. Ltd. The mice were housed under specific pathogen free conditions. They were maintained in a 12 hour light and dark cycle with the temperature at 20~25°C and humidity of 40%~70% and given free access to $Co^{60}$ radiated-sterile diet and autoclaved sterile water. There were four mice in each cage.

**Test article and formulation:** Compound A was dissolved in 0.5% CMC-Na, vortexed for 1-3 min, sonicated for 15 min and stored at 4°C. Compound A was formulated at 2.0, 4.0 and 8.0 mg/mL and prepared once a week.

**Tumor cells inoculation and anti-tumor efficacy study:** When NCI-H716 cells reached about 80-90% in confluence, they were detached by Trypsin-EDTA and collected after centrifugation, then suspended in serum free-medium. Each mouse was injected subcutaneously in the right lateral flank with 0.2 mL of cells suspension containing $5\times10^6$ tumor cells with Martrigel (1:1).

When the NCI-H716 tumors grew to around average 300 $mm^3$, the tumor bearing mice were randomized to vehicle and Compound A treated groups. Compound A in 0.5% CMC-Na as prepared above was orally administered to

mice at 20, 40 and 80 mg/kg twice daily for three weeks, and the vehicle group received 0.5% CMC-Na orally twice daily. The dosing volume was 10 mL/kg body weight. Tumor volume (TV) was measured two times per week by caliper for length and width, and calculated using the formula: $TV = width^2 \times length/2$.

**At** the end of the study, tumors were harvested and tumor growth inhibition (TGI) and tumor weight inhibition ($IR_{TW}$) were calculated by the following equations, where $V_0$ were the data at day 0 ($D_0$) (starting day) and $Vt$ were the data at measurement day t ($D_t$):

$$TGI = [1-(V_t-V_0)_{drug\ treated} / (V_t-V_0)_{vehicle}]\times 100\%$$

$$IR_{TW}\,(\%) = [(TW_{vehicle}-TW_{treatment}) / TW_{vehicle}] \times 100\%$$

**Statistical Analysis:** Student's t-test was used for in comparison with vehicle control. Differences were considered statistically significant at $P < 0.05$.

**Results**

As FIG. 1 indicated, Compound A demonstrated dose dependent anti-tumor effect in NCI-H716 model. At the end of study, H716 tumor growth was repressed by 57.1%, 73.9% and 80.0% at Compound A 20, 40 and 80 mg/kg bid, respectively. And Compound A also decreased tumor weight by over 60% at middle and high doses. Statistically significant difference was seen when Compound A treated group was compared with vehicle treated group (Table 7). No clinical signs of toxicity were observed in Compound A treated mice.

Table 7. Anti-tumor efficacy summary of Compound A in NCI-H716 tumor model

| Group | TGI (%) | $IR_{TW}$ (%) |
|---|---|---|
| Compound A-20, bid | 57.1* | 49.9* |
| Compound A-40, bid | 73.9** | 62.6** |
| Compound A-80, bid | 80.0** | 69.8** |
| *, $P<0.05$; **, $P<0.01$ *vs* vehicle treated group | | |

NCI-H716 xenograft could be considered as a neuroendocrine carcinoma. Compound A inhibited NCI-H716 growth in a dose dependent manner, indicating that Compound A would have therapeutic potential for the treatment of neuroendocrine carcinomas or neuroendocrine tumors in clinic.

**Example 3: Cell viability assay on NCI-H716**

**Cell line**

NCI-H716: human cecum colorectal adenocarcinoma cell line, purchased from ATCC (American Type Culture Collection).
Culture medium: RPMI 1640 containing 10% FBS.

**Materials and solution**

Compound: Compound A (lot#100223, Compound purity of 96.7%) was synthesized in Hutchison Medipharma Limited;
CCK-8 kit (Cell Counting Kit-8): Brand: Dojindo, Catalog No.CK04-01; Microplate reader: Model: Labsystems Multiskan K3, Brand: Thermo.

**Cells treatment**

NCI-H716 cells were diluted with RPMI-1640 medium containing 10% FBS. 90 μL of the diluted cells were added into each well of a 96-well plate so that each well contained $1\times10^4$ cells. The cells were subsequently incubated at 37°C, 5% $CO_2$ overnight.

Test compound dissolved in DMSO was diluted to 30, 10, 3.33, 1.11, 0.37, 0.12, 0.04, 0.01μM with serum free RPMI-1640 medium containing 5% DMSO. Then 10 μL of the diluted compound was added into the above 90 μL cell cultures (final concentration of DMSO in the reaction mixture should be 0.5%), and incubated at 37°C, 5% $CO_2$ for 72 h.

**CCK-8 assay**

10μL of CCK-8 solution was added into cells, and incubated at 37°C, 5% $CO_2$ for additional 1h. The optical density of each well was measured at 450 nm and 630 nm, respectively, by Labsystems Multiskan K3.

**Data analysis**

$$\text{Inhibition}(\%) = 100 - \frac{[\text{OD compound}]_{450\text{-}630} - [\text{OD background}]_{450\text{-}630}}{[\text{OD cell}]_{450\text{-}630} - [\text{OD background}]_{450\text{-}630}} \text{ X } 100$$

$[\text{OD compound}]_{450\text{-}630}$: the optical density of well containing cells with compound treatment;
$[\text{OD cell}]_{450\text{-}630}$: the optical density of wells containing cells without compound treatment;
$[\text{OD background}]_{450\text{-}630}$: the optical density of wells containing culture medium only.

$IC_{50}$: $IC_{50}$ is calculated based on the inhibition curve fitted by XL-Fit 2.0 software.

Result: $IC_{50}$ of Compound A on inhibition of NCI-H716 cells viability was determined as 0.159 μM.

**Example 4: Pharmacokinetics of Compound A in Beagle Dogs Following a Single Oral Administration of Two Different Capsule Formulations Comprising Compound A**

This dog PK study included two periods, with a crossover study design and a washout period of 1 week. Twelve beagle dogs were divided to two groups, 3 males and 3 females for each group. The dosage was 30 mg/kg, one capsule each dog. In period 1, Group 1 was administered Capsule A (micronized Compound A formulation, 50 mg Capsule No. 8 prepared in Example 1), and Group 2 was administered Capsule B (non-micronized Compound A formulation). Capsule B was prepared by the manner described for 50 mg Capsule No. 8 above. Each Capsule A and Capsule B contained 50 mg Compound A. The difference between Capsule A and Capsule B is that Compound A in Capsule A is micronized Form I of Compound A with D90=8.1μm whereas Compound A in Capsule B is non-micronized Form I of Compound A with D90=39.2 μm. The accumulative release of Compound A was 98.1% from 50mg Capsule No.8 and 75.1% from Capsule B at 30min.

After one week's washout, Group 1 was administered Capsule B and Group 2 was administered Capsule A.

Blood samples were collected at different time points after dosing in each period. The detailed information of grouping and time points for blood collection is shown in Table 8.

Table 8 Grouping and time points for collection of blood samples

| Period | Dosing Date | Group No. | Test article | Sample Collection | |
|---|---|---|---|---|---|
| | | | | Sample | Time points |
| 1 | 24-July-2012 | 1 | Capsule A | Plasma | 0, 15, 30 min and 1, 1.5, 2, 3, 4, 5, 6, 8, 10, 12, 24h |
| | | 2 | Capsule B | Plasma | |
| 2 | 31-July-2012 | 1 | Capsule B | Plasma | |
| | | 2 | Capsule A | Plasma | |

The method of protein precipitation with acetonitrile containing phenacetin (internal standard, IS) was used for the dog plasma pretreatment. Compound A concentrations in plasma were determined by a liquid chromatography-tandem mass spectrometry (LC-MS/MS) under a gradient elution condition with Mobile Phase A (deionized water containing 0.1% formic acid) and Mobile Phase B (acetonitrile containing 0.1% formic acid). Chromatographic conditions used are shown in Table 9.

Table 9 Chromatographic conditions

| | | | |
|---|---|---|---|
| Column temperature | 25°C | | |
| Injection volume | 10μL | | |
| Acquisition time | 3.5min | | |
| Autosampler temperature | Approx. 8°C | | |
| Autosampler loop size | 20 μL | | |
| Time | Flow (μL/min) | Mobile phase A | Mobile phase B |
| 0 min | 500 | 95% | 5% |
| 2.00 min | 500 | 10% | 90% |
| 2.10 min | 500 | 95% | 5% |
| 3.50 min | 500 | 95% | 5% |

**Sample Analysis**

Symmetry C18 column (2.1×50 mm, 3.5μm) was used for the sample analysis. Multiple reaction monitoring (MRM) and the positive mode with electrospray ionization (ESI) were applied, and the corresponding detection ions (Q1/Q3) of Compound A were 481.2/329.3. PK parameters were calculated using the non-compartment analysis of Kinetica software. The bioequivalence for the two capsules was evaluated through the statistic tests on the exposure of Compound A in plasma with the confidence interval method and on the Tmax of Compound A in plasma with the Friedman rank sum test.

A. Preparation of stock solution and working solutions

Compound A powder (23.68 mg) was weighed and dissolved in 942 μL of DMSO. Vortex and ultrasonication were performed until the powder was dissolved completely. Compound A primary stock solution was obtained at 25 mg/mL. Compound A stock solution (40 μL) was spiked into 960 μL acetonitrile. After vortex, Compound A secondary stock solution was obtained at 1.0 mg/mL.

The preparation of working solutions for calibration standard (C) and quality control (QC) is shown in Table 10 and Table 11, respectively.

Table 10 Preparation of working solutions for calibration curve (C)

| Source solution (ng/mL) | Took volume (μL) | Added volume of acetonitrile (μL) | Working solution concentration (ng/mL) | Code |
|---|---|---|---|---|
| **1,000,000** | 100 | 900 | 100,000 | |
| **100.000** | 200 | 800 | 20,000 | C1 |
| **20,000** | 500 | 500 | 10,000 | C2 |
| **10,000** | 400 | 600 | 4,000 | C3 |
| **4,000** | 250 | 750 | 1,000 | C4 |
| **1,000** | 400 | 600 | 400 | C5 |
| **400** | 500 | 500 | 200 | C6 |
| **200** | 500 | 500 | 100 | C7 |
| **100** | 500 | 500 | 50 | C8 |

Table 11 Preparation of working solutions for quality control (QC)

| Source solution (ng/mL) | Took volume (μL) | Added volume of acetonitrile (μL) | Working solution concentration (ng/mL) | Code |
|---|---|---|---|---|
| **1,000,000** | 100 | 900 | 100,000 | |
| **100.000** | 160 | 840 | 16,000 | QC5 |
| **16,000** | 150 | 650 | 3,000 | QC4 |
| **3,000** | 100 | 650 | 400 | QC3 |
| **400** | 400 | 400 | 200 | QC2 |
| **200** | 400 | 400 | 100 | QC1 |

B. Preparation of calibration curve and quality control samples

Calibration standard (C) or quality control (QC) working solution (10 μL) was spiked into 190 μL of plasma from naive dogs. After vortex for 1 min, the spiked plasma (50 μL) was transferred into a blank 1.5 mL tube. 150 μL of acetonitrile containing 500 ng/mL phenacetin (internal standard, IS) was added for protein precipitation. The tube was vortexed for 2 min, and then centrifuged in 14000 rpm at 4°C for 10 min. The supernatant (150 μL) was transferred into a 1.5 mL blank tube, and then 150 μL of deionized water was added. After vortex for 1 min, one aliquot of the final solution (10 μL) was used for analysis.

Blank sample was the same as the calibration curve samples except for acetonitrile instead of compound working solutions.

Double blank sample was prepared in the same as blank sample, except that the added acetonitrile solution did not contain phenacetin.

C. Preparation of plasma samples

Plasma sample (50 μL each) obtained from the animal experiment was transferred into a 1.5 mL blank tube, and 150 μL of acetonitrile containing 500 ng/mL phenacetin was added for protein precipitation. After vortex for 2 min, the mixture was centrifuged (14000 rpm at 4 °C for 10 min). The supernatant (150 μL) was transferred into a 1.5 mL blank tube, and then 150 μL of deionized water was added. After vortex for 1 min, one aliquot of the final solution (10 μL) was injected to LC-MS/MS system for analysis.

**Data Analysis**

The peak areas of Compound A and IS were integrated by software Analyst 1.4.1. Standard curves were obtained from standard samples by plotting the peak area ratios of Compound A to IS against the theoretical concentrations of Compound A. The regression curve of Compound A was linear with weighting coefficient of $1/x^2$. The theoretical concentrations of Compound A in calibration standards were 2.50, 5.0, 10, 20, 50, 200, 500 and 1000 ng/mL, respectively. The concentrations of Compound A in dog plasma samples were determined using the standard curves. Pharmacokinetic parameters were calculated based on the plasma concentration-time data, using Thermo Kinetica® software (Version 4.4.1, Thermo Electron Corporation). Non-compartment analysis was performed. The bioequivalence of the two capsules was evaluated through the statistic tests on the exposure of Compound A in dog plasma with confidence interval method and on the Tmax of Compound A in dog plasma with Friedman rank sum test.

**Results**

The PK profile of Compound A in each dog is shown in FIG. 2-FIG. 5. The average plasma concentrations of Compound A plotted against the time points are shown in FIG. 6.

The actual dosages for dogs 3#, 4#, 6#, 7#, 8# and 10# would be lower than designed according to the occurrence time of vomiting and the appearance of vomitus, so the PK results of these dogs were not used for the mean value calculation. The mean values of main Compound A PK parameters in dogs are showed in Table 12.

Table 12 Major PK parameters mean values of Compound A in dogs (n=6)

| Parameter | Unit | Capsule A | | Capsule B | |
|---|---|---|---|---|---|
| | | Mean | SD | Mean | SD |
| Dosage | mg/kg | 30 | - | 30 | - |
| $T_{1/2}$ | h | 5.65 | 1.37 | 5.54 | 1.20 |
| mean residence time (MRT) | h | 11.1 | 1.46 | 10.6 | 1.61 |
| $AUC_{0-24}$ | h·ng/mL | 6753 | 2901 | 4644 | 3619 |
| $AUC_{0-\infty}$ | h·ng/mL | 7388 | 3249 | 4976 | 3802 |
| $C_{max}$ | ng/mL | 623 | 254 | 425 | 367 |
| $T_{max}$ | h | 6.00 | 1.10 | 4.50 | 2.88 |
| Note: the PK parameters of dogs 3#, 4#, 6#, 7#, 8# and 10# were not used for mean calculation due to the emesis after dosing. | | | | | |

Based on the PK profiles of Compound A and the cage-side observations, Capsule A had smaller inter-subject variability and less emesis cases than Capsule B. Compared with Capsule B, Capsule A had higher AUC value after a single oral administration in dogs. There was no significant statistic difference in Tmax mean value in beagle dogs for Capsule A and Capsule B.

## Claims

1. A pharmaceutical composition, comprising
micronized Compound A, and/or
micronized at least one pharmaceutically acceptable salt of Compound A, and
at least one pharmaceutically acceptable excipient,
wherein the micronized Compound A and/or the micronized at least one pharmaceutically acceptable salt of Compound A has a particle size distribution (PSD) D90 value of less than or equal to 20 μm,
wherein Compound A is *N*-(2-(dimethylamino)ethyl)-1-(3-((4-((2-methyl-1*H*-indo1-5-yl)oxy)pyrimidin-2-yl)amino)phenyl)methanesulfonamide represented by the formula:

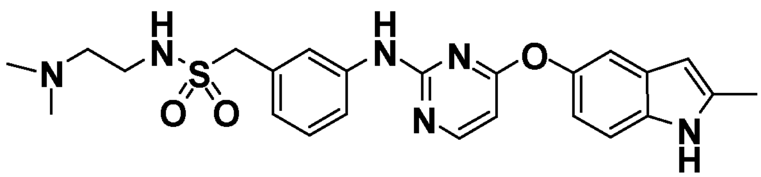

.

2. A pharmaceutical composition of claim 1, comprising at least one pharmaceutically acceptable excipient and micronized Compound A having a D90 value of less than or equal to 20 μm,
wherein Compound A is *N*-(2-(dimethylamino)ethyl)-1-(3-((4-((2-methyl-1*H*-indo1-5-yl)oxy)pyrimidin-2-yl)amino)phenyl)methanesulfonamide represented by the formula:

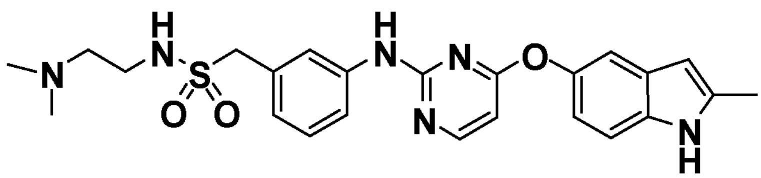

3. The pharmaceutical composition of claim 1 or 2, wherein the micronized Compound A is micronized Form I *N*-(2-(dimethylamino)ethyl)-1-(3-((4-((2-methyl-1*H*-indol-5-yl)oxy)pyrimidin-2-yl)amino)phenyl)methanesulfonamide.

4. The pharmaceutical composition of claim 1 or 2, wherein the micronized Compound A is micronized substantially pure Form I *N*-(2-(dimethylamino)ethyl)-1-(3-((4-((2-methyl-1*H*-indol-5-yl)oxy)pyrimidin-2-yl)amino)phenyl)methanesulfonamide.

5. The pharmaceutical composition of claim 1 or 2, wherein the micronized Compound A is micronized Form II *N*-(2-(dimethylamino)ethyl)-1-(3-((4-((2-methyl-1*H*-indol-5-yl)oxy)pyrimidin-2-yl)amino)phenyl)methanesulfonamide.

6. The pharmaceutical composition of claim 1 or 2, wherein the micronized Compound A is micronized substantially pure Form II N-(2-(dimethylamino)ethyl)-1-(3-((4-((2-methyl-1H-indol-5-yl)oxy)pyrimidin-2-yl)amino)phenyl)methanesulfonamide.

7. The pharmaceutical composition of any one of claims 1-6, wherein the D90 value is less than or equal to 11.0 μm.

8. The pharmaceutical composition of any one of claims 1-6, wherein the D90 value is less than or equal to 4.0 μm.

9. The pharmaceutical composition of any one of claims 1-8, wherein the at least one pharmaceutically acceptable excipient is chosen from diluents, disintegrants, granulation binders, and glidants.

10. The pharmaceutical composition of claim 9, wherein the diluent is selected from mannitol, microcrystalline cellulose, starch, lactose, dextrin and sorbitol, the disintegrant is sodium starch glycolate, the granulation binder is polyvinylpyrrolidone, and the glidant is selected from silicon dioxide and magnesium stearate.

11. Micronized Compound A, and/or micronized at least one pharmaceutically acceptable salt of Compound A, wherein the micronized Compound A and/or the micronized at least one pharmaceutically acceptable salt of Compound A has a particle size distribution (PSD) D90 value of less than or equal to 20 μm,
wherein Compound A is *N*-(2-(dimethylamino)ethyl)-1-(3-((4-((2-methyl-1*H*-indol-5-yl)oxy)pyrimidin-2-yl)amino)phenyl)methanesulfonamide represented by the formula:

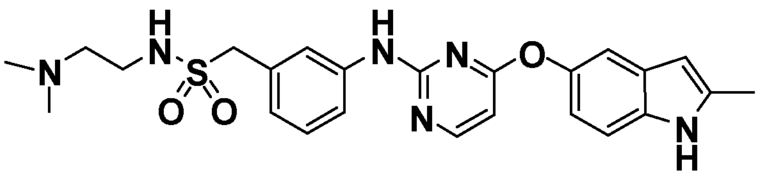

12. Micronized Compound A of claim 11, having a D90 value of less than or equal to 20 μm,
wherein Compound A is *N*-(2-(dimethylamino)ethyl)-1-(3-((4-((2-methyl-1*H*-indol-5-yl)oxy)pyrimidin-2-yl)amino)phenyl)methanesulfonamide represented by the formula:

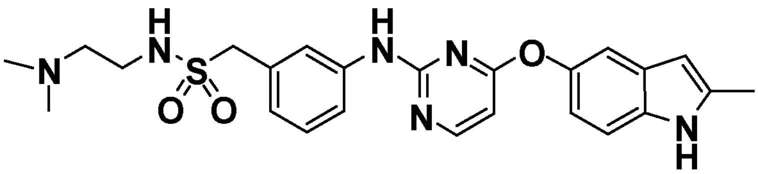

13. The micronized Compound A of claim 12, wherein the micronized Compound A is micronized Form I *N*-(2-(dimethylamino)ethyl)-1-(3-((4-((2-methyl-1*H*-indol-5-yl)oxy)pyrimidin-2-yl)amino)phenyl)methanesulfonamide.

14. The micronized Compound A of claim 12, wherein the micronized Compound A is micronized substantially pure Form I *N*-(2-(dimethylamino)ethyl)-1-(3-((4-((2-methyl-1*H*-indol-5-yl)oxy)pyrimidin-2-yl)amino)phenyl)methanesulfonamide.

15. The micronized Compound A of claim 12, wherein the micronized Compound A is micronized Form II *N*-(2-(dimethylamino)ethyl)-1-(3-((4-((2-methyl-1*H*-indol-5-yl)oxy)pyrimidin-2-yl)amino)phenyl)methanesulfonamide.

16. The micronized Compound A of claim 12, wherein the micronized Compound A is micronized substantially pure

Form II N-(2-(dimethylamino)ethyl)-1-(3-((4-((2-methyl-1H-indol-5-yl)oxy)pyrimidin-2-yl)amino)phenyl)methanesulfonamide.

**17.** The micronized Compound A of any one of claims 11-16, wherein the D90 value is less than or equal to 11.0 μm.

**18.** The micronized Compound A of any one of claims 11-16, wherein the D90 value is less than or equal to 4.0 μm.

**19.** The pharmaceutical composition according to any one of claims 1-9 or the micronized Compound A according to any one of claims 11-18 for use in the treatment of at least one disease responsive to FGFR1 inhibition, and/or at least one disease responsive to KDR inhibition, wherein said disease is an angiogenesis- related disease.

**20.** The pharmaceutical composition according to any one of claims 1-9 or the micronized Compound A according to any one of claims 11-18 for use according to claim 19, wherein said angiogenesis- related disease is selected from the group consisting of cancer and cancer and age-related macular degeneration.

**21.** The pharmaceutical composition according to any one of claims 1-9 or the micronized Compound A according to any one of claims 11-18 for use according to claim 20, wherein said cancer is selected from the group consisting of lung cancer, head and neck cancer, colorectal cancer, pancreatic cancer, colon cancer, breast cancer, ovarian cancer, prostate cancer, stomach cancer, kidney cancer, liver cancer, brain cancer, bone cancer, thyroid cancer, neuroendocrine tumors, sarcoma, and leukemia.

**22.** The pharmaceutical composition according to any one of claims 1-9 or the micronized Compound A according to any one of claims 11-18 for use according to claim 21, wherein said sarcoma is soft tissue sarcoma.

**23.** The pharmaceutical composition according to any one of claims 1-9 or the micronized Compound A according to any one of claims 11-18 for use according to claim 21, wherein said neuroendocrine tumor is pancreatic neuroendocrine tumor or extrapancreatic neuroendocrine tumor.

**24.** The pharmaceutical composition according to any one of claims 1-9 or the micronized Compound A according to any one of claims 11-18 for use according to claim 21, wherein said neuroendocrine tumor is gastrointestinal neuroendocrine tumor.

**Patentansprüche**

**1.** Eine pharmazeutische Zusammensetzung umfassend
die mikronisierte Verbindung A und/oder
das mikronisierte, wenigstens eine pharmazeutisch verträgliche Salz der Verbindung A und
wenigstens einen pharmazeutisch verträglichen Hilfsstoff,
wobei die mikronisierte Verbindung A und/oder das mikronisierte, wenigstens eine pharmazeutisch verträgliche Salz der Verbindung A einen D90-Wert der Teilchengrößenverteilung (PSD) von weniger als oder gleich 20 μm aufweist,
wobei die Verbindung A *N*-(2-(Dimethylamino)ethyl)-1-(3-(4-((2-methyl-1*H*-indol-5-yl)oxy)pyrimidin-2-yl)amino)phenyl)methansulfonamid, dargestellt durch die Formel:

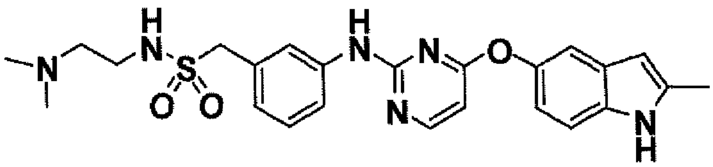

,

ist.

**2.** Eine pharmazeutische Zusammensetzung gemäß Anspruch 1 umfassend wenigstens einen pharmazeutisch verträglichen Hilfsstoff und die mikronisierte Verbindung A, die einen D90-Wert von weniger als oder gleich 20 μm aufweist,

wobei die Verbindung A *N*-(2-(Dimethylamino)ethyl)-1-(3-((4-((2-methyl-1*H*-indol-5-yl)oxy)pyrimidin-2-yl)amino)phenyl)methansulfonamid, dargestellt durch die Formel

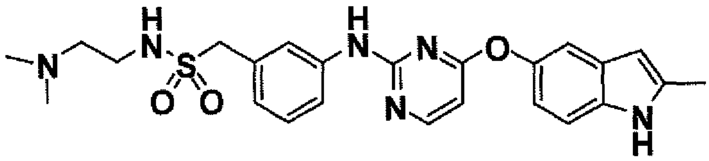

,

ist.

3. Die pharmazeutische Zusammensetzung gemäß Anspruch 1 oder 2, wobei die mikronisierte Verbindung A mikronisiertes *N*-(2-(Dimethylamino)ethyl)-1-(3-((4-((2-methyl-1*H*-indol-5-yl)oxy)pyrimidin-2-yl)amino)phenyl)methansulfonamid der Form I ist.

4. Die pharmazeutische Zusammensetzung gemäß Anspruch 1 oder 2, wobei die mikronisierte Verbindung A mikronisiertes, im Wesentlichen reines *N*-(2-(Dimethylamino)ethyl)-1-(3-((4-((2-methyl-1*H*-indol-5-yl)oxy)pyrimidin-2-yl)amino)phenyl)methansulfonamid der Form I ist.

5. Die pharmazeutische Zusammensetzung gemäß Anspruch 1 oder 2, wobei die mikronisierte Verbindung A mikronisiertes *N*-(2-(Dimethylamino)ethyl)-1-(3-((4-((2-methy)-1*H*-indol-5-yl)oxy)pyrimidin-2-yl)amino)phenyl)methansulfonamid der Form II ist.

6. Die pharmazeutische Zusammensetzung gemäß Anspruch 1 oder 2, wobei die mikronisierte Verbindung A mikronisiertes, im Wesentlichen reines N-(2-(Dimethy)amino)ethyl)-1-(3-((4-((2-methy)-1*H*-indol-5-yl)oxy)pyrimidin-2-yl)amino)phenyl)methansulfonamid der Form II ist.

7. Die pharmazeutische Zusammensetzung gemäß einem der Ansprüche 1-6, wobei der D90-Wert kleiner oder gleich 11,0 μm ist.

8. Die pharmazeutische Zusammensetzung gemäß einem der Ansprüche 1-6, wobei der D90-Wert kleiner oder gleich 4,0 μm ist.

9. Die pharmazeutische Zusammensetzung gemäß einem der Ansprüche 1-8, wobei der wenigstens eine pharmazeutisch verträgliche Hilfsstoff ausgewählt wird aus Verdünnungsmitteln, Sprengmitteln, Granulierbindemitteln und Gleitmitteln.

10. Die pharmazeutische Zusammensetzung gemäß Anspruch 9, wobei das Verdünnungsmittel ausgewählt wird aus Mannitol, mikrokristalliner Cellulose, Stärke, Lactose, Dextrin und Sorbitol, das Sprengmittel Natriumstärkeglycolat ist, das Granulierbindemittel Polyvinylpyrrolidon ist, und das Gleitmittel ausgewählt wird aus Siliciumdioxid und Magnesiumstearat.

11. Eine mikronisierte Verbindung A, und/oder das mikronisierte, wenigstens eine pharmazeutisch verträgliche Salz der Verbindung A,
wobei die mikronisierte Verbindung A und/oder das mikronisierte, wenigstens eine pharmazeutisch verträgliche Salz der Verbindung A einen D90-Wert der Teilchengrößenverteilung (PSD) von weniger als oder gleich 20 μm aufweist,
wobei die Verbindung A *N*-(2-(Dimethylamino)ethyl)-1-(3-((4-((2-methyl-1*H*-indol-5-yl)oxy)pyrimidin-2-yl)amino)phenyl)methansulfonamid, dargestellt durch die Formel:

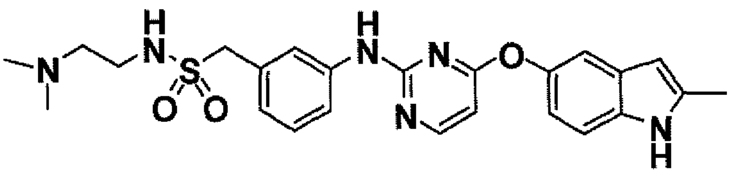

,

ist.

**12.** Eine mikronisierte Verbindung A gemäß Anspruch 11, die einen D90-Wert von weniger als oder gleich 20 µm aufweist,
wobei die Verbindung A *N*-(2-(Dimethylamino)ethyl)-1-(3-((4-((2-methyl-1*H*-indo)-5-yl)oxy)pyrimidin-2-yl)amino)phenyl)methansulfonamid, dargestellt durch die Formel:

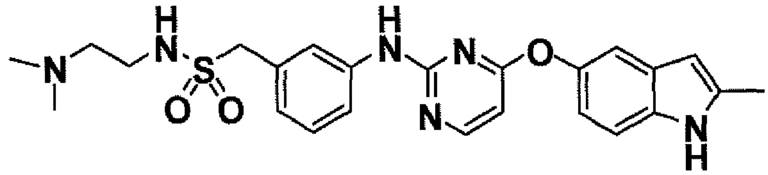,

ist.

**13.** Die mikronisierte Verbindung A gemäß Anspruch 12, wobei die mikronisierte Verbindung A mikronisiertes *N*-(2-(Dimethylamino)ethyl)-1-(3-((4-((2-methyl-1*H*-indol-5-yl)oxy)pyrimidin-2-yl)amino)phenyl)methansulfonamid der Form I ist.

**14.** Die mikronisierte Verbindung A gemäß Anspruch 12, wobei die mikronisierte Verbindung A mikronisiertes, im Wesentlichen reines *N*-(2-(Dimethy)amino)ethyl)-1-(3-((4-(2-methyl-1*H*-indol-5-yl)oxy)pyrimidin-2-yl)amino)phenyl)methansulfonamid der Form I ist.

**15.** Die mikronisierte Verbindung A gemäß Anspruch 12, wobei die mikronisierte Verbindung A mikronisiertes *N*-(2-(Dimethylamino)ethyl)-1-(3-((4-((2-methy[-1H-indol-5-yl)oxy)pyrimidin-2-yl)amino)phenyl)methansulfonamid der Form II ist.

**16.** Die mikronisierte Verbindung A gemäß Anspruch 12, wobei die mikronisierte Verbindung A mikronisiertes, im Wesentlichen reines *N*-(2-(Dimethylamino)ethyl)-1-(3-((4-((2-methyl-1*H*-indol-5-yl)oxy)pyrimidin-2-yl)amino)phenyl)methansulfonamid der Form II ist.

**17.** Die mikronisierte Verbindung A gemäß einem der Ansprüche 11-16, wobei der D90-Wert weniger als oder gleich 11,0 µm ist.

**18.** Die mikronisierte Verbindung A gemäß einem der Ansprüche 11-16, wobei der D90-Wert weniger als oder gleich 4,0 µm ist.

**19.** Die pharmazeutische Zusammensetzung gemäß einem der Ansprüche 1-9 oder die mikronisierte Verbindung A gemäß einem der Ansprüche 11-18 zur Verwendung in der Behandlung von mindestens einer auf FGFR1-Hemmung ansprechenden Krankheit und/oder mindestens einer auf KDR-Hemmung ansprechenden Krankheit, wobei die Krankheit eine mit Angiogenese zusammenhängende Krankheit ist.

**20.** Die pharmazeutische Zusammensetzung gemäß einem der Ansprüche 1-9 oder die mikronisierte Verbindung A gemäß einem der Ansprüche 11-18 zur Verwendung gemäß Anspruch 19, wobei die mit Angiogenese zusammenhängende Krankheit aus der Gruppe bestehend aus Krebs und altersbedingter Makuladegeneration ausgewählt wird.

**21.** Die pharmazeutische Zusammensetzung gemäß einem der Ansprüche 1-9 oder die mikronisierte Verbindung A gemäß einem der Ansprüche 11-18 zur Verwendung gemäß Anspruch 20, wobei der Krebs ausgewählt wird aus der Gruppe bestehend aus Lungenkrebs, Kopf- und Halskrebs, kolorektalem Krebs, Bauchspeicheldrüsenkrebs, Dickdarmkrebs, Brustkrebs, Eierstockkrebs, Prostatakrebs, Magenkrebs, Nierenkrebs, Leberkrebs, Hirnkrebs, Knochenkrebs, Schilddrüsenkrebs, neuroendokrinen Tumoren, Sarkom und Leukämie.

**22.** Die pharmazeutische Zusammensetzung gemäß einem der Ansprüche 1-9 oder die mikronisierte Verbindung A gemäß einem der Ansprüche 11-18 zur Verwendung gemäß Anspruch 21, wobei das Sarkom ein Weichteilsarkom ist.

**23.** Die pharmazeutische Zusammensetzung gemäß einem der Ansprüche 1-9 oder die mikronisierte Verbindung A

gemäß einem der Ansprüche 11-18 zur Verwendung gemäß Anspruch 21, wobei der neuroendokrine Tumor ein neuroendokriner Tumor der Bauchspeicheldrüse oder ein extrapankreatischer neuroendokriner Tumor ist.

**24.** Die pharmazeutische Zusammensetzung gemäß einem der Ansprüche 1-9 oder die mikronisierte Verbindung A gemäß einem der Ansprüche 11-18 zur Verwendung gemäß Anspruch 21, wobei der neuroendokrine Tumor ein gastrointestinaler neuroendokriner Tumor ist.

**Revendications**

**1.** Composition pharmaceutique comprenant :

un composé A micronisé, et/ou
au moins un sel pharmaceutiquement acceptable micronisé du composé A; et
au moins un excipient pharmaceutiquement acceptable ;

dans laquelle le composé A micronisé et/ou ledit au moins un sel pharmaceutiquement acceptable micronisé du composé A possède une valeur D90 de distribution granulométrique (PSD) inférieure ou égale à 20 μm ;
dans laquelle le composé A est le *N*-(2-diméthylamino)éthyl)-1-(3-((4-((2-méthyl-1*H*-indol-5-yl)oxy)pyrimidin-2-yl)amino)phényl)méthanesulfonamide répondant à la formule :

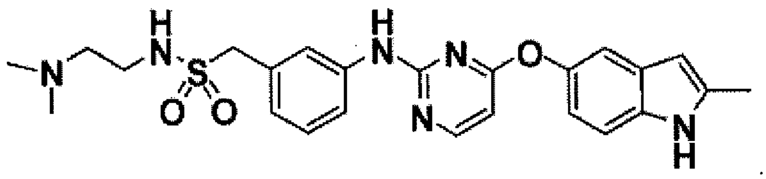

**2.** Composition pharmaceutique selon la revendication 1, comprenant au moins un excipient pharmaceutiquement acceptable et un composé A micronisé dont la valeur D90 est inférieure ou égale à 20 μm ;
dans laquelle le composé A est le *N*-(2-diméthylamino)éthyl)-1-(3-((4-((2-méthyl-1*H*-indo1-5-yl)oxy)pyrimidin-2-yl)amino)phényl)méthanesulfonamide répondant à la formule :

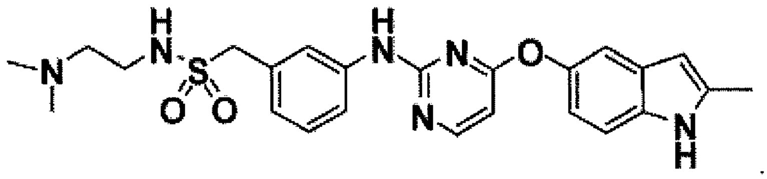

**3.** Composition pharmaceutique selon la revendication 1 ou 2, dans laquelle le composé A micronisé représente la forme **I** micronisée de *N*-(2-diméthylamino)-éthyl)-1-(3-((4-((2-méthyl-1*H*-indol-5-yl)oxy)pyrimidin-2-yl)amino)phényl)-méthanesulfonamide.

**4.** Composition pharmaceutique selon la revendication 1 ou 2, dans laquelle le composé A micronisé représente la forme **I** micronisée essentiellement pure de *N*-(2-diméthylamino)éthyl)-1-(3-((4-((2-méthyl-1*H*-indol-5-yl)oxy)pyrimidin-2-yl)amino)phényl)méthanesuifonamide.

**5.** Composition pharmaceutique selon la revendication 1 ou 2, dans laquelle le composé A micronisé représente la forme **II** micronisée de N-(2-diméthylamino)-éthyl)-1-(3-((4-((2-méthyl-1*H*-indol-5-yl)oxy)pyrimidin-2-yl)amino)phényl)-méthanesulfonamide.

**6.** Composition pharmaceutique selon la revendication 1 ou 2, dans laquelle le composé A micronisé représente la forme **II** micronisée essentiellement pure de *N*-(2-diméthylamino)éthyl)-1-(3-((4-((2-méthyl-1*H*-indol-5-yl)oxy)pyrimidin-2-yl)amino)phényl)méthanesulfonamide.

**7.** Composition pharmaceutique selon l'une quelconque des revendications 1 à 6, dans laquelle la valeur D90 est inférieure ou égale à 11,0 μm.

**8.** Composition pharmaceutique selon l'une quelconque des revendications 1 à 6, dans laquelle la valeur D90 est inférieure ou égale à 4,0 μm.

**9.** Composition pharmaceutique selon l'une quelconque des revendications 1 à 8, dans laquelle ledit au moins un excipient pharmaceutiquement acceptable est choisi parmi des diluants, des délitants, des liants de granulation et des agents de glissement.

**10.** Composition pharmaceutique selon la revendication 9, dans laquelle le diluant est choisi parmi le mannitol, la cellulose microcristalline, l'amidon, le lactose, la dextrine et le sorbitol, le délitant représente le glycolate d'amidon de sodium, le liant de granulation représente la polyvinylpyrrolidone et l'agent de glissement est choisi parmi le dioxyde de silicium et le stéarate de magnésium.

**11.** Composé A micronisé et/ou au moins un sel pharmaceutiquement acceptable micronisé du composé A dans lesquels le composé A micronisé et/ou ledit au moins un sel pharmaceutiquement acceptable micronisé du composé A possèdent une valeur D90 de distribution granulométrique (PSD) inférieure ou égale à 20 μm ;
dans lesquels le composé A est le *N*-(2-diméthylamino)éthyl)-1-(3-((4-((2-méthyl-1*H*-indol-5-yl)oxy)pyrimidin-2-yl)amino)phényl)méthanesulfonamide répondant à la formule :

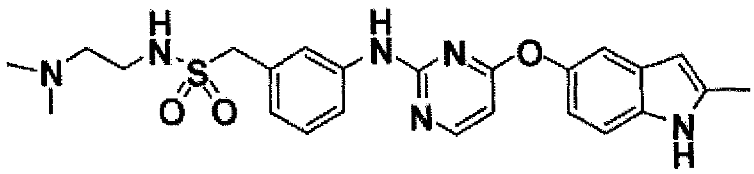

**12.** Composé A micronisé selon la revendication 11, dont la valeur D90 est inférieure ou égale à 20 μm ;
dans lequel le composé A est le *N*-(2-diméthylamino)éthyl)-1-(3-((4-((2-méthyl-1*H*-indol-5-yl)oxy)pyrimidin-2-yl)amino)phényl)méthanesulfonamide répondant à la formule :

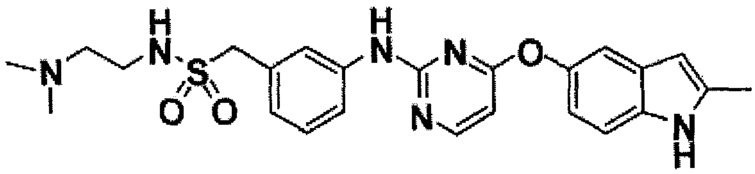

**13.** Composé A micronisé selon la revendication 12, dans lequel le composé A micronisé représente la forme **I** micronisée de *N*-(2-diméthylamino)-éthyl)-1-(3-((4-((2-méthyl-1*H*-indol-5-yl)oxy)pyrimidin-2-yl)amino)phényl)-méthanesulfonamide.

**14.** Composé A micronisé selon la revendication 12, dans lequel le composé A micronisé représente la forme **I** micronisée essentiellement pure de *N*-(2-diméthylamino)éthyl)-1-(3-((4-((2-méthyl-1*H*-indol-5-yl)oxy)pyrimidin-2-yl)amino)phényl)méthanesulfonamide.

**15.** Composé A micronisé selon la revendication 12, dans lequel le composé A micronisé représente la forme **II** micronisée de *N*-(2-diméthylamino)-éthyl)-1-(3-((4-((2-méthyl-1*H*-indol-5-yl)oxy)pyrimidin-2-yl)amino)phényl)-méthanesulfonamide.

**16.** Composé A micronisé selon la revendication 12, dans lequel le composé A micronisé représente la forme **II** micronisée essentiellement pure de *N*-(2-diméthylamino)éthyl)-1-(3-((4-((2-méthyl-1*H*-indol-5-yl)oxy)pyrimidin-2-yl)amino)phényl)méthanesulfonamide.

**17.** Composé A micronisé selon l'une quelconque des revendications 11 à 16, dans laquelle la valeur D90 est inférieure ou égale à 11,0 μm.

**18.** Composé A micronisé selon l'une quelconque des revendications 11 à 16, dans laquelle la valeur D90 est inférieure ou égale à 4,0 μm.

19. Composition pharmaceutique selon l'une quelconque des revendications 1 à 9 ou composé A micronisé selon l'une quelconque des revendications 11 à 18, pour leur utilisation dans le traitement d'au moins une maladie sensible à une inhibition du FGFR1, et/ou d'au moins une maladie sensible à une inhibition du KDR, dans lesquels ladite maladie est une maladie liée à l'angiogenèse.

20. Composition pharmaceutique selon l'une quelconque des revendications 1 à 9 ou composé A micronisé selon l'une quelconque des revendications 11 à 18, pour leur utilisation selon la revendication 19, dans lesquels ladite maladie liée à l'angiogenèse est choisie parmi le groupe constitué par un cancer et une dégénérescence maculaire liée à un cancer et à l'âge.

21. Composition pharmaceutique selon l'une quelconque des revendications 1 à 9 ou composé A micronisé selon l'une quelconque des revendications 11 à 18, pour leur utilisation selon la revendication 20, dans lesquels le cancer en question est choisi parmi le groupe constitué par un cancer du poumon, un cancer de la tête et du cou, un cancer colorectal, un cancer pancréatique, un cancer du côlon, un cancer du sein, un cancer des ovaires, un cancer de la prostate, un cancer de l'estomac, un cancer des reins, un cancer du foie, un cancer du cerveau, un cancer des os, un cancer de la thyroïde, des tumeurs neuroendocrines, un sarcome et une leucémie.

22. Composition pharmaceutique selon l'une quelconque des revendications 1 à 9 ou composé A micronisé selon l'une quelconque des revendications 11 à 18, pour leur utilisation selon la revendication 21, dans lesquels le sarcome en question est un sarcome des tissus mous.

23. Composition pharmaceutique selon l'une quelconque des revendications 1 à 9 ou composé A micronisé selon l'une quelconque des revendications 11 à 18, pour leur utilisation selon la revendication 21, dans lesquels ladite tumeur neuroendocrine est une tumeur neuroendocrine pancréatique ou une tumeur neuroendocrine extrapancréatique.

24. Composition pharmaceutique selon l'une quelconque des revendications 1 à 9 ou composé A micronisé selon l'une quelconque des revendications 11 à 18, pour leur utilisation selon la revendication 21, dans lesquels ladite tumeur neuroendocrine est une tumeur neuroendocrine gastro-intestinale.

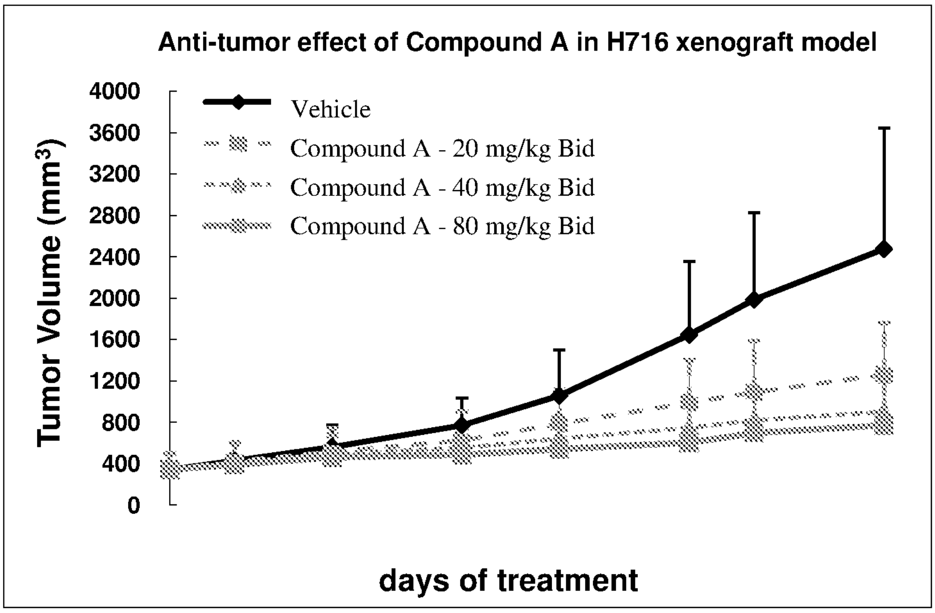
Anti-tumor effect of Compound A in H716 xenograft model
Tumor Volume (mm3)
4000
3600
3200
2800
2400
2000
1600
1200
800
400
0
Vehicle
Compound A - 20 mg/kg Bid
Compound A - 40 mg/kg Bid
Compound A - 80 mg/kg Bid
days of treatment

Figure 1

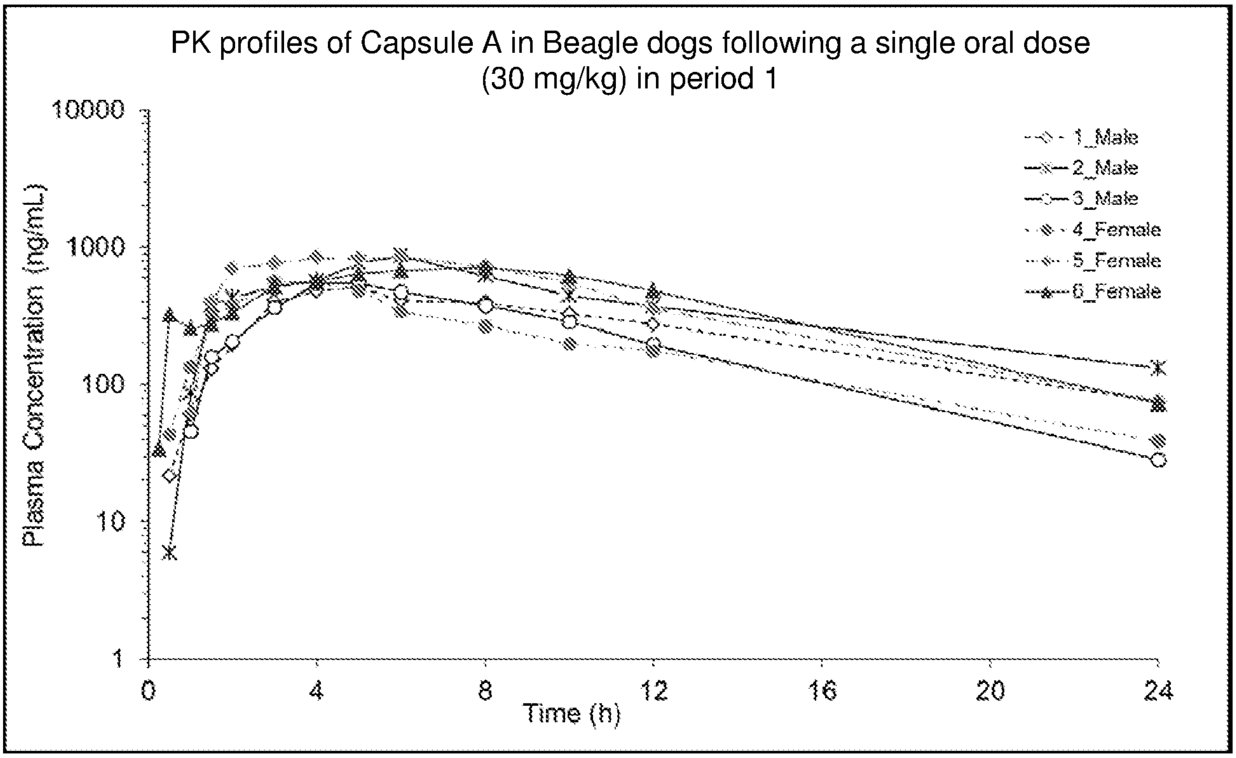
PK profiles of Capsule A in Beagle dogs following a single oral dose
(30 mg/kg) in period 1
Plasma Concentration (ng/mL)
10000
1000
100
10
1
0
4
8
12
16
20
24
Time (h)
1_Male
2_Male
3_Male
4_Female
5_Female
6_Female

Figure 2

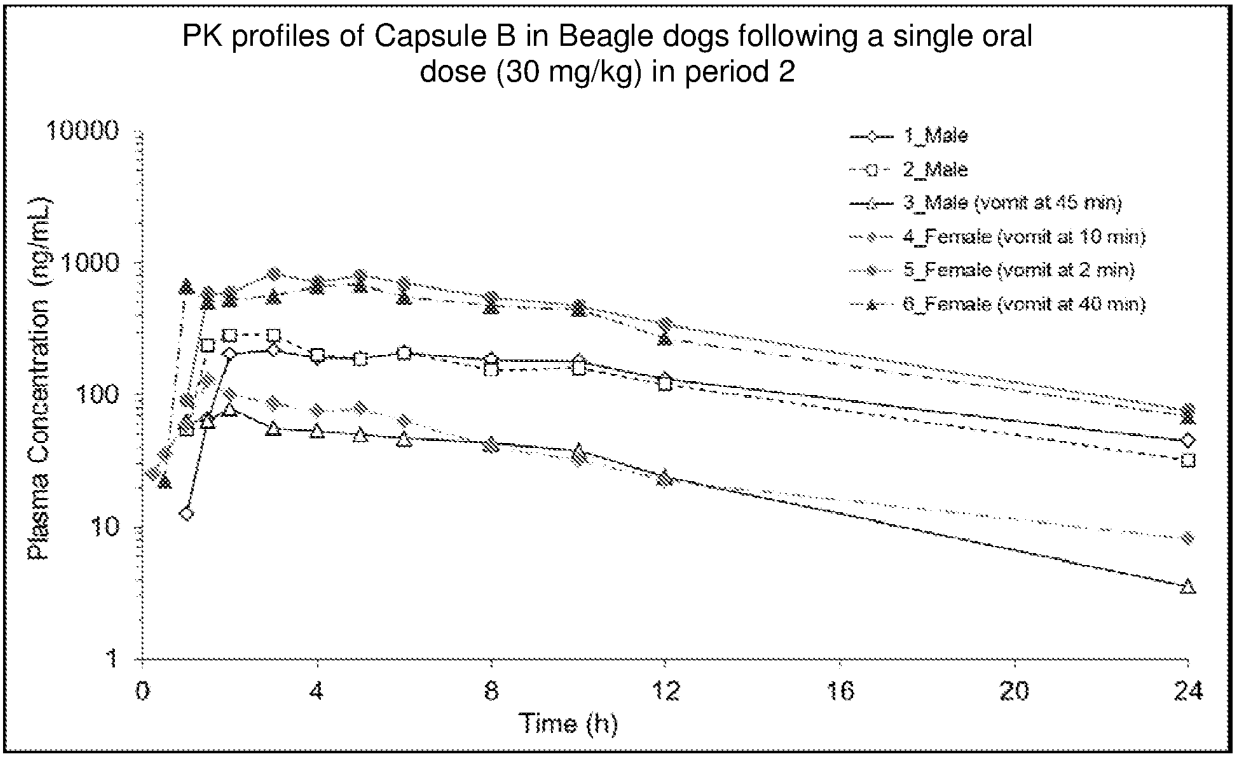
PK profiles of Capsule B in Beagle dogs following a single oral dose (30 mg/kg) in period 2
Plasma Concentration (ng/mL)
10000
1000
100
10
1
0
4
8
12
16
20
24
Time (h)
1_Male
2_Male
3_Male (vomit at 45 min)
4_Female (vomit at 10 min)
5_Female (vomit at 2 min)
6_Female (vomit at 40 min)

Figure 3

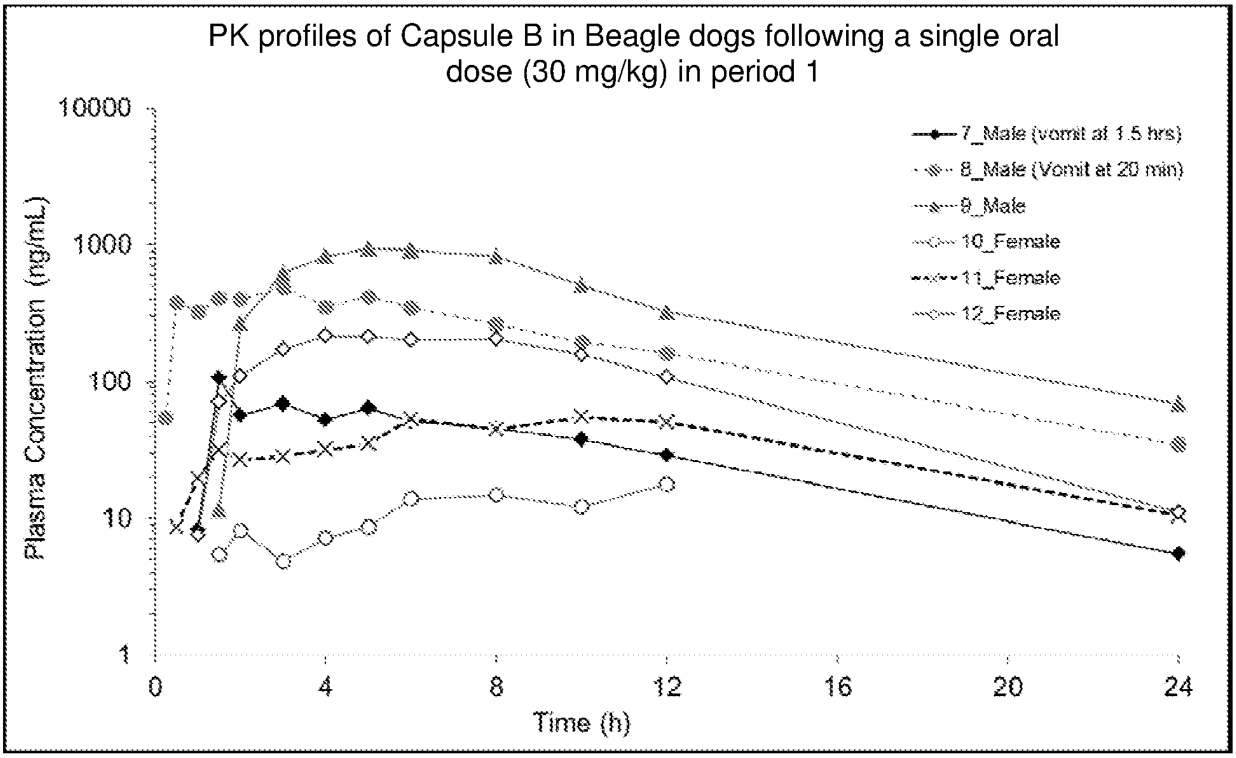
PK profiles of Capsule B in Beagle dogs following a single oral dose (30 mg/kg) in period 1
10000
1000
100
10
1
Plasma Concentration (ng/mL)
0
4
8
12
16
20
24
Time (h)
7_Male (vomit at 1.5 hrs)
8_Male (Vomit at 20 min)
9_Male
10_Female
11_Female
12_Female

Figure 4

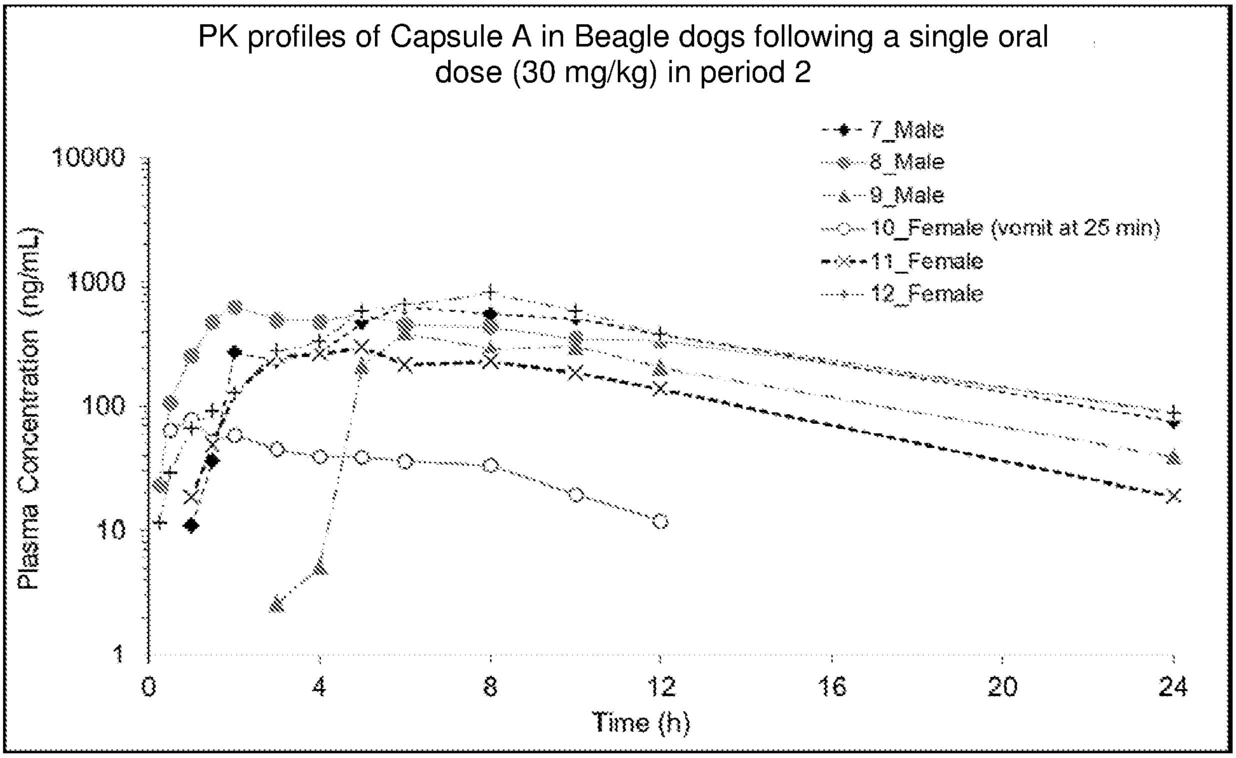
PK profiles of Capsule A in Beagle dogs following a single oral dose (30 mg/kg) in period 2
7_Male
8_Male
9_Male
10_Female (vomit at 25 min)
11_Female
12_Female
10000
1000
100
10
1
Plasma Concentration (ng/mL)
0
4
8
12
16
20
24
Time (h)

Figure 5

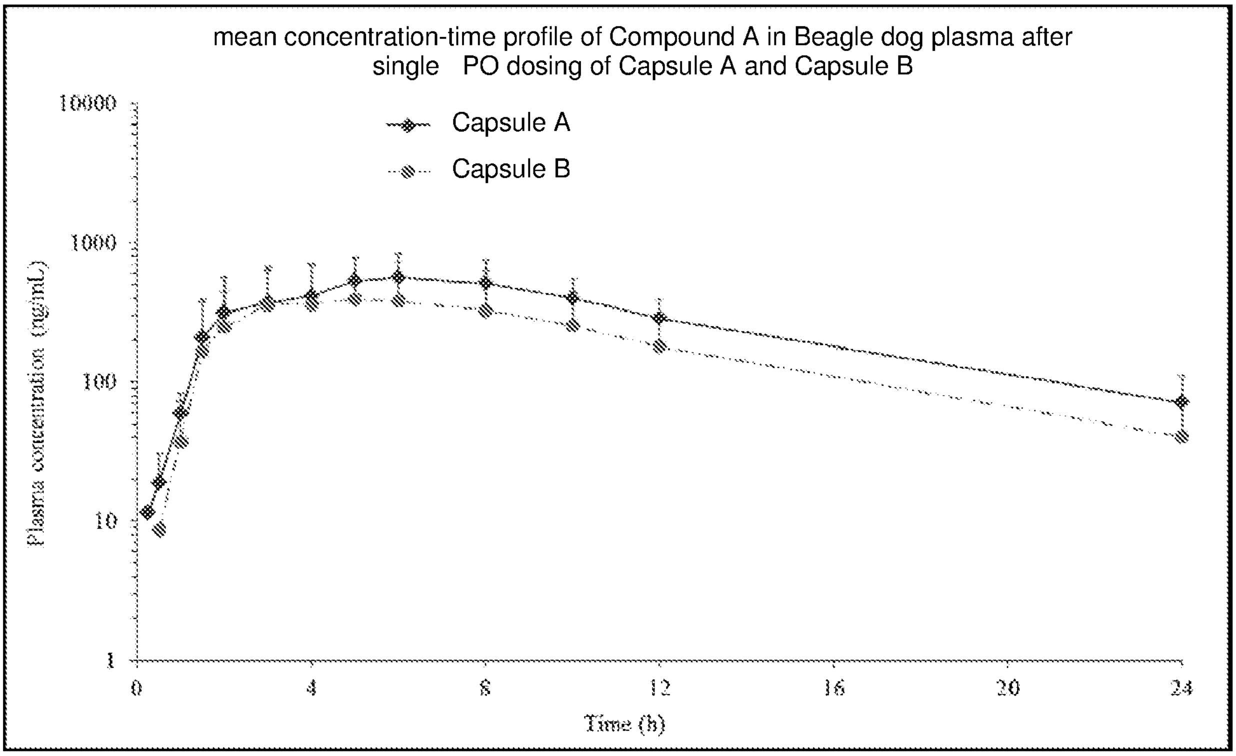
mean concentration-time profile of Compound A in Beagle dog plasma after single PO dosing of Capsule A and Capsule B
Capsule A
Capsule B
10000
1000
100
10
1
Plasma concentration (ng/mL)
0
4
8
12
16
20
24
Time (h)

Figure 6

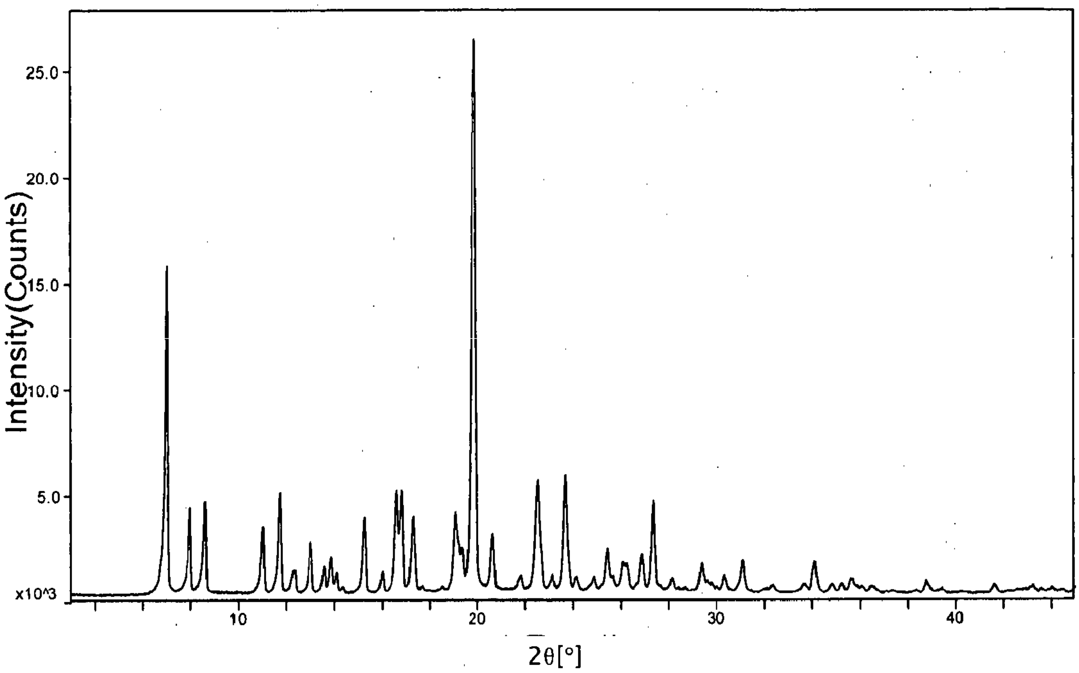


Figure 7

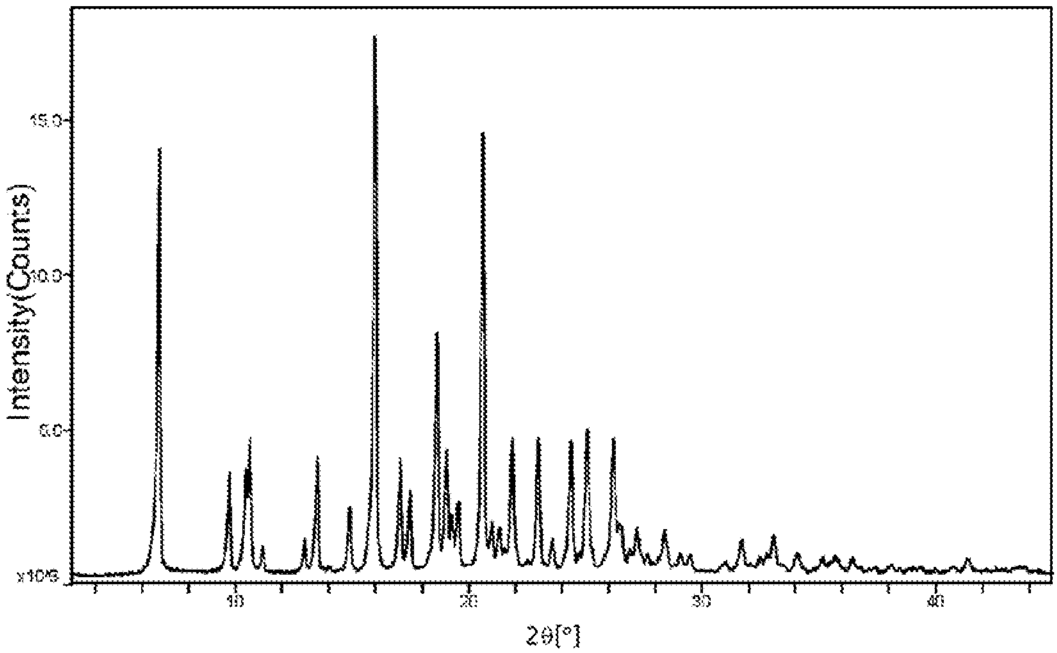


Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 510249
- CN 2010078997 W
- US 8658658 B


### Non-patent literature cited in the description


- **RUBIN M. TUDER.** *Chest,* 2000, vol. 117, 281
- *China Pharmacopoeia*